(19) Europäisches Patentamt
European Patent Office
Office européen des brevets

(11) Veröffentlichungsnummer : **0 138 030**
**B1**

(12) # EUROPÄISCHE PATENTSCHRIFT

(45) Veröffentlichungstag der Patentschrift : **30.09.87**

(51) Int. Cl.⁴ : **C 07 C 91/23**, C 07 C 93/14, C 07 C 97/10, A 61 K 31/135

(21) Anmeldenummer : **84110558.8**

(22) Anmeldetag : **05.09.84**

(54) **Phenäthylamin-Derivate.**

(30) Priorität : **15.09.83 CH 5032/83**
**04.07.84 CH 3231/84**

(43) Veröffentlichungstag der Anmeldung : **24.04.85 Patentblatt 85/17**

(45) Bekanntmachung des Hinweises auf die Patenterteilung : **30.09.87 Patentblatt 87/40**

(84) Benannte Vertragsstaaten :
**AT BE CH DE FR GB IT LI LU NL SE**

(56) Entgegenhaltungen :
**EP-A- 0 112 669**
**FR-A- 2 304 331**
**FR-M- 6 408**

(73) Patentinhaber : **F. HOFFMANN-LA ROCHE & CO. Aktiengesellschaft**
**CH-4002 Basel (CH)**

(72) Erfinder : **Bernauer, Karl, Prof. Dr.**
**Wartenbergstrasse 30**
**CH-4104 Oberwil (CH)**
Erfinder : **Bruderer, Hans, Dr.**
**Schulgasse 4**
**CH-4105 Biel-Benken (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 138 030 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Phenäthylamin-Derivate mit wertwollen pharmakodynamischen Eigenschaften. Im speziellen betrifft sie Verbindungen der allgemeinen Formel

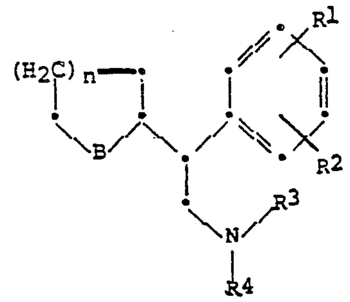

(I)

worin $R^1$ und $R^2$ je Wasserstoff, Halogen, Trifluormethyl, niederes Alkoxy, niederes Alkyl, Hydroxy oder Nitro bedeuten, wobei mindestens einer von $R^1$ und $R^2$ von Wasserstoff verschieden ist, $R^3$ und $R^4$ je niederes Alkyl, n die Zahl, 1, 2, 3 oder 4 und B die Gruppe —CO— oder —CHOH— bedeuten, und pharmazeutisch annehmbare Säureadditionssalze davon.

Gegenstand der vorliegenden Erfindung sind die Verbindungen der obigen Formel I und ihre pharmazeutisch annehmbaren Säureadditionssalze als solche und als pharmazeutische Wirkstoffe, die Herstellung dieser Verbindungen und Zwischenprodukte für deren Herstellung, Arzneimittel, enthaltend eine solche Verbindung, und die Herstellung solcher Arzneimittel, sowie die Verwendung dieser Verbindungen bei der Bekämpfung oder Verhütung von Krankheiten.

Der Ausdruck « nieder » in Kombinationen, wie « niederes Alkyl », « niedere Alkylgruppe », « niederes Alkoxy » und dergleichen, bezeichnet Reste mit höchstens 4 Kohlenstoffatomen. Der Ausdruck « Alkyl » bezeichnet geradkettige oder verzweigte, gesättigte Kohlenwasserstoffreste, wie Methyl, Aethyl, Isopropyl und dergleichen. Der Ausdruck « Alkoxy » bezeichnet über ein Sauerstoffatom gebundene Alkylgruppen, wie z. B. Methoxy, Aethoxy, Isopropoxy und dergleichen. Der Ausdruck « Halogen » bedeutet Fluor, Chlor, Brom oder Jod.

Die Verbindungen der eingangs definierten Formel I besitzen zwei oder, wenn B die Gruppe —CHOH— bedeutet, drei asymmetrisch substituierte Kohlenstoffatome. Die vorliegende Erfindung umfasst sowohl die optisch einheitlichen Formen dieser Verbindungen als auch die verschiedenen diastereoisomeren Racemate und Gemische von unterschiedlichen diastereoisomeren Racematen.

Besonders bevorzugt werden die diastereoisomeren Racemate mit der in der allgemeinen Formel Ia bzw. Ib gezeigten relativen Konfiguration, sowie die entsprechenden optisch einheitlichen enantiomeren Formen :

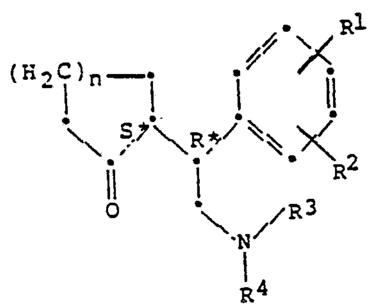

(Ia)

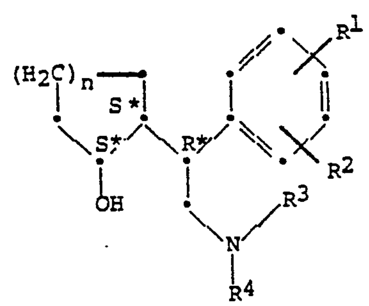

(Ib)

worin $R^1$, $R^2$, $R^3$, $R^4$ und n obige Bedeutung besitzen.

In einer bevorzugten Ausführungsform umfasst die vorliegende Erfindung Verbindungen der Formel I, worin entweder $R^1$ Halogen, niederes Alkoxy oder Hydroxy und $R^2$ Wasserstoff oder $R^1$ und $R^2$ jeweils beide Halogen, niederes Alkoxy oder Hydroxy bedeuten. Vorzugsweise bedeutet der Ausdruck « Halogen » Chlor und der Ausdruck « niederes Alkoxy » Methoxy. Besonders bevorzugt sind diejenigen Verbindungen der Formel I, worin $R^1$ Chlor oder Hydroxy und $R^2$ Wasserstoff, Chlor oder Hydroxy bedeuten, und insbesondere diejenigen, worin $R^2$ Wasserstoff bezeichnet und $R^1$ in 4-Stellung des Phenylringes steht und Chlor oder Hydroxy bedeutet oder $R^1$ in 3-Stellung des Phenylringes steht und Hydroxy bedeutet. Vorzugsweise bedeutet n die Zahl 2. B bedeutet vorzugsweise die Gruppe —CHOH—. $R^3$ und $R^4$ stehen vorzugsweise für Methyl.

Im Rahmen der vorliegenden Erfindung ganz besonders bevorzugte Verbindungen sind :

rac-(1S*)-cis-2-/(R*)-4-Chlor-α-[(dimethylamino)-methyl]benzyl/cyclohexanol und
rac-(1S*)-cis-2-/(R*)-α-[(Dimethylamino)methyl]-4-hydroxybenzyl/cyclohexanol

Weitere besonders bevorzugte Verbindungen sind :

(1S)-cis-2-/(R)-4-Chlor-α-[(dimethylamino)methyl]-benzyl/cyclohexanol,
(1R)-cis-2-/(S)-4-Chlor-α-[(dimethylamino)methyl]-benzyl/cyclohexanol,
rac-(1S*)-cis-2-/(R*)-3,4-Dichlor-α-[(dimethylamino)-methyl]benzyl/cyclohexanol,
rac-(1S*)-cis-2-/(R*)-4-Methoxy-α-[(dimethylamino)-methyl]benzyl/cyclohexanol,
rac-(1S*)-cis-2-/(R*)-α-[(Dimethylamino)methyl]-3,4-dimethoxybenzyl/cyclohexanol,
rac-(2S*)-2/(R*)-4-Chlor-α-[(dimethylamino)methyl]-benzyl/cyclohexanon und
(1S)-cis-2-/(R)-α-[(Dimethylamino)methyl]-4-hydroxy-benzyl/cyclohexanol.

Die Verbindungen der Formel I und ihre pharmazeutisch annehmbaren Säureadditionssalze können erfindungsgemäss dadurch hergestellt werden, dass man

a) in einer Verbindung der allgemeinen Formel

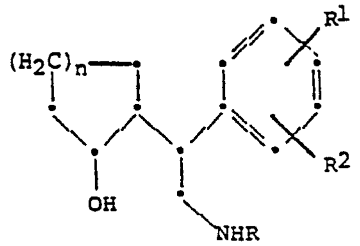

(II)

worin $R^1$, $R^2$ und n obige Bedeutung besitzen, und R Wasserstoff oder niederes Alkyl bedeutet, die primäre oder sekundäre Aminogruppe di- bzw. monoalkyliert, oder

b) in einer Verbindung der allgemeinen Formel

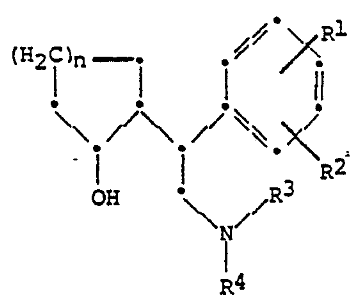

(Ic)

worin $R^1$, $R^2$, $R^3$, $R^4$ und n obige Bedeutung besitzen, die sekundäre Alkoholgruppierung oxidiert, oder

c) aus einer Verbindung der allgemeinen Formel

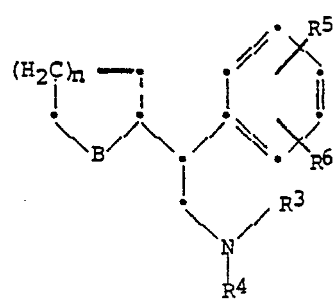

(III)

worin $R^3$, $R^4$, n und B obige Bedeutung besitzen, und einer der Reste $R^5$ und $R^6$ eine geschützte Hydroxylgruppe und der andere Wasserstoff, Halogen, Trifluormethyl, niederes Alkoxy, niederes Alkyl, Nitro oder eine geschützte Hydroxylgruppe bedeutet, die Schutzgruppe (n) abspaltet, oder

d) eine Verbindung der allgemeinen Formel

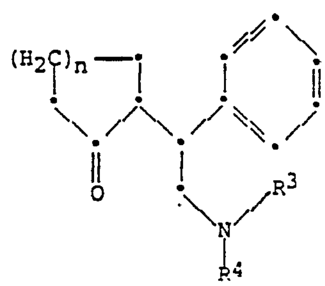

(IV)

worin $R^3$, $R^4$ und n obige Bedeutung besitzen, in der p-Stellung des Phenylringes nitriert, und erwünschtenfalls

e) ein erhaltenes Gemisch von unterschiedlichen Racematen in die Racemate auftrennt,

f) ein erhaltenes Racemat in die optischen Antipoden spaltet und/oder

g) eine erhaltene Verbindung der Formel I in ein pharmazeutisch annehmbares Säureadditionssalz überführt.

Gemäss Verfahren a) werden Verbidungen der Formel I hergestellt, worin B die Gruppe —CHOH— bedeutet, und $R^1$, $R^2$, $R^3$, $R^4$ und n die eingangs erwähnte Bedeutung besitzen. Für die N-Alkylierung kommen verschiedene an sich bekannte und jedem Fachmann geläufige Methoden in Betracht. Man kann beispielsweise eine Verbindung der Formel II in einem inerten organischen Lösungsmittel, wie Dimethylformamid, Acetonitril, Tetrahydrofuran oder dergleichen, und in Gegenwart eines säurebindenden Mittels, wie Kaliumcarbonat, Triäthylamin oder dergleichen, mit einem Alkylhalogenid, wie Methyljodid, oder mit einem Dialkylsulfat, wie Dimethylsulfat, umsetzen. Die Alkylierung kann jedoch auch in zwei Schritten durchgeführt werden. In einem ersten Schritt wird die Verbindung der Formel II mit einem reaktiven Carbonsäurederivat, beispielsweise mit einem Carbonsäurechlorid, umgesetzt, worauf man in einem zweiten Schritt das gebildete Carbonsäureamid mit einem reaktiven komplexen Hydrid, z. B. mit Lithiumaluminiumhydrid, zum entsprechenden Amin reduziert. Diese Methode eignet sich insbesondere für die Alkylierung von Verbindungen der Formel II, worin R niederes Alkyl bedeutet. In einer bevorzugten Ausführungsform verwendet man als Ausgangsmaterial eine Verbindung der Formel II, worin R Wasserstoff bedeutet, und alkyliert diese mit einem Aldehyd, wie Formaldehyd, in Gegenwart eines geeigneten Reduktionsmittels, wie Ameisensäure. Als Lösungsmittel kann man beispielsweise Dimethylformamid, Tetrahydrofuran, 1,2-Dimethoxyäthan oder dergleichen verwenden. Verwendet man Ameisensäure als Reduktionsmittel, so ist ein zusätzliches Lösungsmittel nicht unbedingt erforderlich. Die Temperatur ist nicht kritisch ; die Reaktion kann in einem Bereich von etwa Raumtemperatur bis zur Siedetemperatur der Reaktionsmischung durchgeführt werden.

Gemäss Verfahren b) werden Verbindungen der Formel I hergestellt, worin B die Gruppe —CO— bedeutet, und $R^1$, $R^2$, $R^3$, $R^4$ und n die eingangs erwähnte Bedeutung besitzen. Für die Oxidation der sekundären Alkoholgruppierung in einer Verbindung der Formel Ic können verschiedene bekannte Oxidationsmittel, wie konzentrierte Salpetersäure, Jones-Reagens, Kaliumpermanganat oder dergleichen, verwendet werden, wobei die jeweils üblichen Reaktionsbedingungen angewendet werden. Ein für die Zwecke der vorliegenden Erfindung besonders bevorzugtes Oxidationsmittel ist konzentrierte (65-proz.) Salpetersäure. In diesem Fall ist ein zusätzliches Lösungsmittel nicht notwendig. Die Oxidation mit konzentrierter Salpetersäure wird zweckmässigerweise bei tiefen Temperaturen, d. h. in einem Bereich von etwa —30 °C bis 0 °C, durchgeführt.

Gemäss Verfahren c) werden Verbindungen der Formel I hergestellt, worin $R^1$ und/oder $R^2$ Hydroxy bedeuten, und $R^3$, $R^4$, n und B die eingangs erwähnte Bedeutung besitzen. Als Schutzgruppen eignen sich natürlich nur solche, welche unter den Bedingungen der weiter unten beschriebenen Herstellung der Verbindungen der Formel III stabil sind und welche selektiv entfernt werden können, ohne dass andere im Molekül vorhandene Strukturelemente in Mitleidenschaft gezogen werden. Die Auswahl der hierfür geeigneten Schutzgruppen dürfte dem Fachmann keinerlei Schwierigkeiten bereiten. Besonders gut geeignete Schutzgruppen, welche die obigen Bedingungen erfüllen, sind beispielsweise die Benzylgruppe oder am Phenylring substituierte Benzylgruppen. Solche Schutzgruppen können durch katalytische Hydrierung, beispielsweise in Gegenwart von Palladium/Kohle bei Raumtemperatur und Atmosphärendruck, leicht abgespalten werden.

Gemäss Verfahren d) werden Verbindungen der Formel I hergestellt, worin $R^1$ Nitro in p-Stellung, $R^2$ Wasserstoff und B die Gruppe —CO— bedeuten, und $R^3$, $R^4$ und n die eingangs erwähnte Bedeutung besitzen. Die Nitrierung wird nach an sich bekannten und jedem Fachmann geläufigen Methoden durchgeführt. Für die Zwecke der vorliegenden Erfindung verwendet man vorzugsweise rauchende Salpetersäure als Nitrierungsmittel, wobei in diesem Fall ein zusätzliches Lösungsmittel nicht notwendig ist. Die Nitrierung mit rauchender Salpetersäure wird zweckmässigerweise bei tiefen Temperaturen, d. h. in einem Bereich von etwa — 30 °C bis 0 °C, durchgeführt.

Je nach Stereochemie der eingesetzten Ausgangsstoffe erhält man die gewünschten Produkte als Gemisch von verschiedenen Racematen, als Racemate oder als optisch einheitliche Formen. Gemische von verschiedenen Racematen können mittels herkömmlicher Methoden aufgetrennt werden, wobei sich

die fraktionierte Kristallisation und die Säulenchromatographie an Kieselgel oder Aluminiumoxid besonders gut eignen. Racemate können beispielsweise über die fraktionierte Kristallisation der mit optisch aktiven Säuren, wie (R,R)-Weinsäure, (S,S)-Weinsäure oder dergleichen, erhaltenen diastereoisomeren Ammoniumsalze gespalten werden.

Die Herstellung von pharmazeutisch annehmbaren Säureadditionssalzen von Verbindungen der Formel I erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulphonate, p-Toluolsulfonate, Oxalate, Tartrate und dergleichen.

Die als Ausgangsstoffe verwendeten Verbindungen der Formel II sind neu und ebenfalls Gegenstand der vorliegenden Erfindung. Sie können hergestellt werden, indem man eine Verbindung der allgemeinen Formel

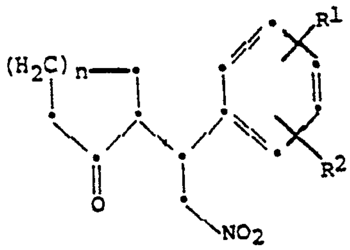
(V)

oder

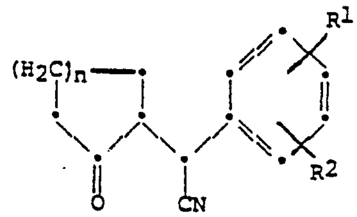
(VI)

worin $R^1$, $R^2$ und n obige Bedeutung besitzen, mit einem reaktiven, komplexen Metallhydrid, wie Lithiumaluminiumhydrid, in einem inerten organischen Lösungsmittel, wie Tetrahydrofuran, reduziert und erwünschtenfalls in der erhaltenen Verbindung der allgemeinen Formel

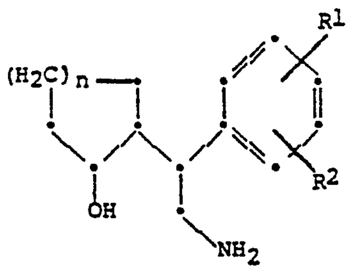
(IIa)

worin $R^1$, $R^2$ und n obige Bedeutung besitzen, die primäre Aminogruppe mono-alkyliert. Diese Alkylierung wird zweckmässigerweise so durchgeführt, dass man die Verbindung der Formel IIa zuerst mit einem reaktiven Carbonsäurederivat, beispielsweise einem Carbonsäurechlorid, acyliert und dann das gebildete Amid mit einem reaktiven, komplexen Metallhydrid, wie Lithiumaluminiumhydrid, zum gewünschten sekundären Amin reduziert. Erwünschtenfalls können erhaltene Gemische von diastereoisomeren Racematen von Verbindungen der Formel II bereits auf dieser Stufe aufgetrennt werden, wobei sich die fractionierte Kristallisation und die Säulenchromatographie an Kieselgel oder Aluminiumoxid hierfür gut eignen. Natürlich können auch allfällig erhaltene Racemate bereits auf dieser Stufe gespalten werden. Andererseits kann man, und dies gilt insbesondere für die Verbindungen der Formel V, bereits Ausgangsstoffe mit definierter Stereochimie für die Herstellung von Verbindungen der Formel II verwenden.

Die Verbindungen der Formel V sind bekannt oder können in Analogie zu den bekannten Vertretern dieser Stoffklasse dadurch hergestellt werden, dass man eine Verbindung der allgemeinen Formel

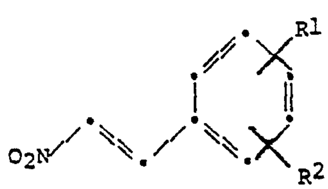
(VII)

worin $R^1$ und $R^2$ obige Bedeutung besitzen, mit einem Enamin oder Lithiumenolat eines ($C_{5-8}$)-Cycloalkanons umsetzt. Bei der Verwendung von ($C_{5-8}$)-Cycloalkanon-Enaminen erhält man als Produkt ein Enamin einer Verbindung der Formel V, das dann zum Keton der Formel V hydrolysiert wird. Verwendet man bei dieser Reaktion ein optisch aktives Enamin, das durch Umsetzen eines optisch aktiven sekundären Amins, wie (R)-2-Methoxymethyl-pyrrolidin, mit einem ($C_{5-8}$)-Cycloalkanon erhalten werden kann, so können optisch aktive Verbindungen der Formel V in hoher optischer Ausbeute erhalten werden.

Die Verbindungen der Formel VI können hergestellt werden, indem man in an sich bekannter Weise eine Verbindung der allgemeinen Formel

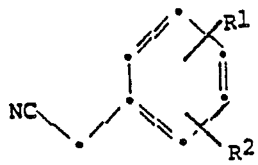

(VIII)

worin $R^1$ und $R^2$ obige Bedeutung besitzen, in Gegenwart einer starken Base mit einer Verbindung der allgemeinen Formel

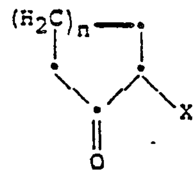

(IX)

worin X ein Halogenatom bedeutet, und n obige Bedeutung besitzt, umsetzt.

Die Verbindungen der Formel III sind neu und ebenfalls Gegenstand der vorliegenden Erfindung. Sie können hergestellt werden, indem man in an sich bekannter Weise in einer Verbindung der allgemeinen Formel

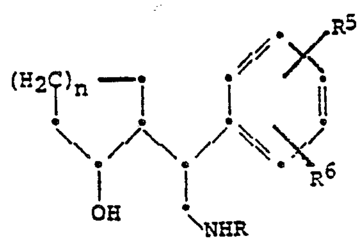

(X)

worin $R^5$, $R^6$, R und n obige Bedeutung besitzen, die primäre oder sekundäre Aminogruppe di- bzw. monoalkyliert und gewünschtenfalls die sekundäre Alkoholgruppierung in der erhaltenen Verbindung oxidiert. Die Alkylierung wird vorzugsweise mit einem Aldehyd, wie Formaldehyd, in Gegenwart eines Reduktionsmittels, wie Ameisensäure, und die Oxidation vorzugsweise mit konzentrierter Salpetersäure (65-proz.) durchgeführt.

Die Verbindungen der Formel X können gleich hergestellt werden wie die Verbindungen der Formel II, und zwar ausgehend von Verbindungen der Formel VII oder VIII, worin jedoch $R^1$ und $R^2$ die oben für $R^5$ und $R^6$ gegebenen Bedeutungen haben.

Die Verbindungen der Formel IV sind neu und ebenfalls Gegenstand der vorliegenden Erfindung. Sie können hergestellt werden, indem man in an sich bekannter Weise in einer Verbindung der allgemeinen Formel

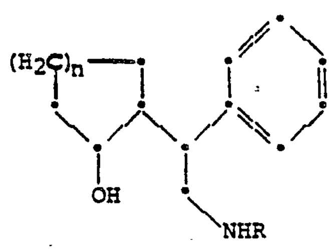

(XI)

worin R und n obige Bedeutung besitzen, die primäre oder sekundäre Aminogruppe di- bzw. monoalkyliert und anschliessend die sekundäre Alkoholgruppierung in der erhaltenen Verbindung oxidiert. Die Alkylierung wird vorzugsweise mit einem Aldehyd, wie Formaldehyd, in Gegenwart eines Reduktionsmittels, wie Ameisensäure, und die Oxidation vorzugsweise mit konzentrierter Salpetersäure (65-proz.) durchgeführt.

Die Verbindungen der Formel XI können gleich hergestellt werden wie die Verbindungen der Formel II, und zwar ausgehend von Verbindungen der Formel VII oder VIII, worin jedoch $R^1$ und $R^2$ Wasserstoff bedeuten.

Wie bereits eingangs erwähnt, besitzen die Verbindungen der Formel I und ihre pharmazeutisch annehmbaren Säureadditionssalze wertvolle pharmakodynamische Eigenschaften. Sie besitzen insbesondere interessante analgetische, antidepressive und akut entzündungshemmende Eigenschaften. Die analgetische Wirkung kann im bekannten und von der Fachwelt anerkannten Writhing-Test, an der Maus, und die antidepressive Wirkung kann im bekannten Serotonin-uptake-Test [vgl. M.J. Kuhar et al., Journal of Pharmacology and Experimental Therapeutics 181, 36 (1972)] gezeigt werden. In der nachfolgenden Tabelle werden die in diesen Tests ermittelten Resultate für einige repräsentative Vertreter der durch die allgemeine Formel I definierten Verbindungsklasse angegeben. Die Tabelle enthält ausserdem Angaben über die akute Toxizität dieser Verbindungen ($DL_{50}$ nach einmaliger oraler Verabreichung an Mäusen).

Tabelle

| Verbindung der Formel I | Writhing-Test $ED_{50}$ in mg/kg p.o. | Serotonin-uptake-Test, $IC_{50}$ in nM | $DL_{50}$ in mg/kg p.o. |
|---|---|---|---|
| rac-(1S*)-cis-2-/(R*)-4-Chlor-α-[(dimethylamino)methyl]benzyl/cyclohexanol-Hydrochlorid | 18 | 88 | 312-625 |
| rac-(1S*)-cis-2-/(R*)-α-[(Dimethylamino)-methyl]-4-hydroxybenzyl/cyclohexanol-Hydrochlorid | 8,4 | 180 | 500-1000 |
| (1S)-cis-2-/(R)-4-Chlor-α-[(dimethyl-amino)methyl]benzyl/cyclohexanol-Hydrochlorid | 14 | 110 | 125-250 |
| (1R)-cis-2-/(S)-4-Chlor-α-[(dimethylamino)-methyl]benzyl/cyclohexanol-Hydrochlorid | 19 | 55 | 125-250 |
| rac-(1S*)-cis-2-/(R*)-3,4-Dichlor-α-[(dimethylamino)methyl]benzyl/cyclohexanol-Hydrochlorid | 9,7 | 21 | 62,6-125 |
| rac-(1S*)-cis-2-/(R*)-4-Methoxy-α-[(dimethylamino)methyl]benzyl/cyclohexanol-Hydrochlorid | 23 | 120 | 250-500 |
| rac-(1S*)-cis-2-/(R*)-α-[(Dimethylamino)-methyl]-3,4-dimethoxybenzyl/cyclohexanol-Hydrochlorid | 10 | 100 | 500-1000 |
| rac-(2S*)-2-/(R*)-4-Chlor-α-[(dimethylamino)-methyl]benzyl/cyclohexanon-Hydrochlorid | 22 | 240 | 156-312 |

Wie eingangs erwähnt, kann man Verbindungen der allgemeinen Formel I und pharmazeutisch annehmbare Säureadditionssalze davon erfindungsgemäss bei der Bekämpfung oder Verhütung von Krankheiten verwenden, insbesondere bei der Bekämpfung oder Verhütung von Schmerzen und Depressionen. Die Dosierung der Verbindungen der Formel I und ihrer pharmazeutisch annehmbaren Säureadditionssalze kann dabei innerhalb weiter Grenzen variieren und ist natürlich in jedem einzelnen Fall den individuellen Gegebenheiten anzupassen. Im allgemeinen dürften bei oraler Verabreichung eine Tagesdosis von 25-150 mg angemessen sein.

Die Verbindungen der Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von pharmazeutischen Präparaten können die erfindungsgemässen Produkte mit pharmazeutisch bzw. therapeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen ; je nach Beschaffenheit des Wirkstoffes sind jedoch bei Weichgelatinekapseln übehaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze, zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe in eine galenische Darreichungsform bringt.

Die nachfolgenden Beispiele sollen die vorliegende Erfindung näher erläutern : ihren Umfang jedoch in keiner Weise beschränken. Sämtliche Temperaturen sind in Celsiusgraden angegeben. Der Ausdruck « Aether » steht für Diäthyläther und der Ausdruck « Isopropyläther » für Diisopropyläther.

Beispiel 1

a) Zu einer Lösung von 215,7 g (1,29 Mol) N-(1-cyclohexen-1-yl)morpholin in 600 ml Methylenchlorid wird unter Rühren und Kühlen eine Lösung von 237 g (1,29 Mol) 4-Chlor-ω-nitrostyrol in 1 400 ml Methylenchlorid innert etwa 2 Stunden so zugetropft, dass die Temperatur 5° nicht übersteigt. Anschliessend wird während 3 Stunden weitergerührt. Unter intensivem Rühren versetzt man mit Eis und dann rasch mit 710 ml 2N-Salzsäurelösung, worauf man noch während 30 Minuten rührt. Die wässrige Phase wird abgetrennt und zweimal mit je 1 l Methylenchlorid extrahiert, die organische Phase wird zweimal mit je 500 ml Wasser gewaschen, über Magnesiumsulfat getrocknet und auf etwa 300 ml eingeengt. Nach Zugabe von 2 l Aether wird über Nacht im Eisschrank stehen gelassen. Man erhält rac-(2S*)-2-[(R*)-4-Chlor-α-(nitromethyl)benzyl] cyclohexanon in Form farbloser Kristalle vom Schmelzpunkt 96-97°.

b) Zu einer Suspension von 39 g (1,03 Mol) Lithiumaluminiumhydrid in 1 l trockenem Tetrahydrofuran wird unter Argon eine Lösung von 112 g (0,4 Mol) rac-(2S*)-2-[(R*)-4-Chlor-α-(nitromethyl)benzyl] cyclohexanon in 1 l trockenem Tetrahydrofuran so zugetropft, dass die Temperatur 50° nicht übersteigt, worauf das Reaktionsgemisch über Nacht bei 50° gerührt wird. Nach dem Abkühlen auf Raumtemperatur behandelt man das Reaktionsgemisch mit 50 ml Aethanol und anschliessend mit 200 ml Tetrahydrofuran/Wasser (1 : 1), saugt den ausgefallenen Niederschlag unter Waschen mit Methylenchlorid ab und dampft das Filtrat ein. Das erhaltene Oel wird in 1 l Aether gelöst, und die Lösung wird über Nacht bei Raumtemperatur stehen gelassen. Die ausgefallenen farblosen Kristalle werden abgesaugt, wobei man rac-(1S*)-cis-2-[(R*)-α-(Aminomethyl)-4-chlorbenzyl] cyclohexanol vom Smp. 108-110° erhält. Das aus der Base auf übliche Weise bereitete rac-(1S*)-cis-2-[(R*)-α-(Aminomethyl)-4-chlorbenzyl] cyclohexanol-Hydrochlorid wird aus Aethanol/Aether umkristallisiert und schmilzt bei 259-260° (Zersetzung).

c) Eine Lösung von 37,7 g (0,15 Mol) rac-(1S*)-cis-2-[(R*)-α-(Aminomethyl)-4-chlorbenzyl] cyclohexanol in 300 ml Dimethylformamid wird mit 30 ml 37-proz. Formaldehydlösung und 15 ml 88-proz.

Ameisensäure versetzt und während 3 Stunden bei 100° gehalten. Nach dem Abkühlen wird der leichtflüchtige Teil bei 75° im Wasserstrahlvakuum abdestilliert, der Rückstand wird mit 3N-Natronlauge versetzt, worauf man mit Methylenchlorid extrahiert. Die organische Phase wird mit Wasser gewaschen, über Magnesiumsulfat getrocknet und eingedampft. Das erhaltene farblose Oel wird in Hexan aufgenommen. Nach Stehenlassen über Nacht bei Raumtemperatur wird der ausgefallene Niederschlag abgesaugt. Man erhält rac-(1S*)-cis-2-/(R*)-4-Chlor-α-[(dimethylamino) methyl] benzyl/cyclohexanol in Form farbloser Kristalle vom Smp. 88-89°. Das aus der Base bereitete rac-(1S*)-cis-2-/(R*)-4-Chlor-α-[(diethylamino) methyl] benzyl/cyclohexanol-Hydrochlorid wird aus Aethanol/Aether umkristallisiert. Man erhält farblose Kristalle vom Smp. 267-268°.

Beispiel 2

20,0 g (0,07 Mol) rac-(1S*)-cis-2-/(R*)-4-Chlor-α-[(dimethylamino) methyl] benzyl/cyclohexanol und 5,83 g (0,04 Mol) natürliche (R,R)-Weinsäure werden zusammen in 500 ml heissem Aethanol gelöst, und die Lösung wird über Nacht bei Raumtemperatur stehengelassen. Das ausgefallene (+)-(1S)-cis-2-/(R)-4-Chlor-α-[(dimethylamino) methyl] benzyl/cyclohexanol-(R,R)-tartrat (1 : 1) wird aus 100 ml Methanol umgelöst, wobei farblose Kristalle vom Smp. 178-179° (Zersetzung) anfallen ; $\alpha_D = +27°$ (c = 1, Wasser). Man löst das erhaltene Tartrat in Wasser, stellt mit 3N-Natronlauge alkalisch und extrahiert die Base mit Aether. Die organische Phase wird einmal mit Wasser gewaschen, über Magnesiumsulfat getrocknet und eingedampft. Das erhaltene kristalline Produkt wird aus Isopropyläther/Hexan kristallisiert. Man erhält (—)-(1S)-cis-2-/(R)-4-Chlor-α-[(dimethylamino) methyl] benzyl/cyclohexanol vom Smp. 117-117,5° ; $\alpha_D = -7{,}9°$ (c = 1, Aethanol). Das aus der Base auf übliche Art bereitete (+)-(1S)-cis-2-/(R)-4-Chlor-α-[(dimethylamino) methyl] benzyl/cyclohexanol-Hydrochlorid wird aus Aethanol/Aether umkristallisiert um schmilzt bei 278-279° ; $\alpha_D = +24{,}4°$ (c = 1, Wasser).

Beispiel 3

Das in Beispiel 2 nach Abtrennen des kristallinen R,R-Tartrats erhaltene Filtrat wird eingedampft, der Rückstand wird mit 5,83 g unnatürlicher (S,S)-Weinsäure versetzt, und das Gemisch wird in 100 ml Methanol in der Siedehitze gelöst. Nach dem Abkühlen wird mit 50 ml Aether versetzt und über Nacht bei Raumtemperatur stehengelassen. Die erhaltenen farblosen Kristalle werden aus 100 ml heissem Methanol umgelöst. Man erhält (—)-(1R)-cis-2-/(S)-4-Chlor-α-[(dimethylamino) methyl] benzyl/cyclohexanol-(S,S)-tartrat vom Smp. 177-177,5° ; $\alpha_D = -26{,}3°$ (c = 1, Wasser). Das daraus bereitete (+)-(1R)-cis-2-/(S)-4-Chlor-α-[(dimethylamino) methyl] benzyl/cyclohexanol wird aus Isopropyläther umkristallisiert und schmilzt bei 115-116° ; $\alpha_D = +8{,}2°$ (c = 1, Aethanol). Das auf übliche Weise bereitete (—)-(1R)-cis-2-/(S)-4-Chlor-α-[(dimethylamino) methyl] benzyl/cyclohexanol-Hydrochlorid wird aus Aethanol/Aether umkristallisiert und hat einen Smp. von 278-279° ; $\alpha_D = -23{,}6°$ (c = 1, Wasser).

Beispiel 4

a) Zu einer Lösung von 130,3 g (0,78 Mol) N-(1-Cyclohexen-1-yl) morpholin in 800 ml Methylenchlorid unter Stickstoff wird unter Rühren und Kühlen eine Lösung von 143 g (0,78 Mol) 2-Chlor-ω-nitrostyrol in 800 ml Methylenchlorid innert etwa 1,5 Stunden so zugetropft, das die Temperatur 20° nicht übersteigt. Anschliessend wird noch während 3 Stunden gerührt. Unter intensivem Rühren wird mit 408 ml 2N-Salzsäure versetzt und während 30 Minuten gerührt. Die wässrige Phase wird abgetrennt und zweimal mit je 500 ml Methylenchlorid extrahiert, die organische Phase wird mit Wasser gewaschen, über Magnesiumsulfat getrocknet und eingedampft. Der ölige Rückstand wird mit 500 ml Isopropyläther versetzt, worauf man über Nacht im Eisschrank stehen lässt. Man erhält rac-(2S*)-2-[(R*)-2-Chlor-α-(nitromethyl) benzyl] cyclohexanon in Form farbloser Kristalle vom Smp. 107-108°.

b) Zu einer Suspension von 18,9 g (0,5 Mol) Lithium-aluminiumhydrid in 500 ml trockenem Tetrahydrofuran unter Argon wird unter Rühren eine Lösung von 56,3 g (0,2 Mol) rac-(2S*)-2-[(R*)-2-Chlor-α-(nitromethyl) benzyl] cyclohexanon in 500 ml trockenem Tetrahydrofuran so zugetropft, dass die Temperatur 50° nicht übersteigt. Das Reaktionsgemisch wird dann noch während 15 Stunden bei 50° gerührt. Nach dem Abkühlen versetzt man zuerst mit 50 ml Aethanol und anschliessend mit Tetrahydrofuran/Wasser (1 : 1), saugt den ausgefallenen Niederschlag unter Waschen mit Methylenchlorid ab und dampft das Filtrat ein. Der erhaltene ölige Rückstand wird mit 3N-Salzsäure versetzt, worauf man zweimal mit je 250 ml Aether extrahiert. Die wässrige Phase wird mit 3N-Natronlauge alkalisch gestellt und zweimal mit je 500 ml Methylenchlorid extrahiert. Die organische Phase wird mit Wasser gewaschen, über Magnesiumsulfat getrocknet und eingedampft. Man erhält das Rohprodukt in Form eines gelben Oels ; es wird auf übliche Weise in das Hydrochlorid übergeführt. Nach mehrmaligem Umkristallisieren aus Methylenchlorid/Methanol erhält man rac-(1S*)-cis-2-[(R*)-α-(Aminomethyl)-2-chlorbenzyl] cyclohexanol-Hydrochlorid vom Smp. 278-280°.

c) 7,6 g (30 mMol) rac-(1S*)-cis-2-[(R*)-α-(Aminomethyl)-2-chlorbenzyl] cyclohexanol werden in

60 ml Dimethylformamid gelöst, worauf man mit 30 ml 37-proz. Formaldehydlösung und 15 ml 88-proz. Ameisensäure versetzt und während 5 Stunden auf 100° erwärmt. Nach dem Abdestillieren der leichtflüchtigen Anteile bei 70° im Wasserstrahlvakuum wird der Rückstand mit 3N-Natronlauge versetzt. Man extrahiert die Base zweimal mit je 500 ml Aether. Die organische Phase wird mit Wasser gewaschen, über Magnesiumsulfat getrocknet und eingedampft. Das erhaltene gelbe Oel wird an der zehnfachen Menge Aluminiumoxid (Aktivitätsstufe II, neutral) chromatographiert. Durch Eluieren mit 500 ml Toluol/Aether (1 : 1) erhält man ein farbloses Oel, das nach Behandeln mit Hexan kristallisiert. Das erhaltene rac-(1S*)-cis-2-/(R*)-2-Chlor-α-[(dimethylamino) methyl] benzyl/cyclohexanol schmilzt bei 60-61°. Das daraus bereitete rac-(1S*)-cis-2-/(R*)-2-Chlor-α-[(dimethylamino) methyl] benzyl/cyclohexanol-Hydrochlorid wird aus Essigester umkristallisiert, wobei man farblose Kristalle vom Smp. 180-180,5°, erhält.

Beispiel 5

a) Zu einer Lösung von 40,1 g (0,24 Mol) N-(1-Cyclohexen-1-yl) morpholin in 400 ml Methylenchlorid unter Stickstoff wird unter Rühren und Kühlen eine Lösung von 52,3 g (0,24 Mol) 3,4-Dichlor-ω-nitrostyrol in 500 ml Methylenchlorid innert etwa 45 Minuten so zugegeben, dass die Temperatur 0° nicht übersteigt. Anschliessend wird noch während 3 Stunden bei 0° gerührt. Unter intensivem Rühren werden 420 ml 1N-Salzsäure rasch zugegeben, worauf man noch während 30 Minuten rührt. Die wässrige Phase wird abgetrennt und zweimal mit je 500 ml Methylenchlorid extrahiert ; die organische Phase wird mit Wasser gewaschen, über Magnesiumsulfat getrocknet und eingedampft. Man nimmt den erhaltenen öligen Rückstand in 250 ml Isopropyläther auf und lässt über Nacht im Eisschrank stehen. Man erhält rac-(2S*)-2-([(R*)-3,4-Dichlor-α-(nitromethyl) benzyl] cyclohexanon in Form farbloser Kristalle vom Smp. 109-110°.

b) Zu einer Suspension von 8,53 g (0,22 Mol) Lithium-aluminiumhydrid in 200 ml trockenem Tetrahydrofuran unter Argon wird eine Lösung von 28,5 g (0,09 Mol) rac-(2S*)-2-[(R*)-3,4-Dichlor-α-(nitromethyl) benzyl] cyclohexanon in 500 ml trockenem Tetrahydrofuran zo zugetropft, dass die Temperatur 50° nicht übersteigt, worauf man während 15 Stunden bei 50° rührt. Nach dem Abkühlen behandelt man das Reaktionsgemisch mit Tetrahydrofuran/Wasser (1 : 1), saugt den ausgefallenen Niederschlag unter Waschen mit Methylenchlorid ab und dampft das Filtrat ein. Das so erhaltene Oel wird mit 3N-Salzsäure versetzt, worauf man zweimal mit je 500 ml Aether extrahiert. Man stellt die wässrige Phase mit 3N-Natronlauge alkalisch und extrahiert zweimal mit je 500 ml Methylenchlorid. Die organische Phase wird zweimal mit je 100 ml Wasser gewaschen, über Magnesiumsulfat getrocknet und eingedampft. Man erhält ein zähes Oel, das aus Isopropyläther/Hexan kristallisiert. Man erhält rac-(1S*)-cis-2-[(R*)-α-(Aminomethyl)-3,4-dichlorbenzyl] cyclohexanol in Form farbloser Kristalle vom Smp. 117-118°.

c) Man löst 7,2 g (25 mMol) rac-(1S*)-cis-2-[(R*)-α-(Aminomethyl)-3,4-dichlorbenzyl] cyclohexanol in 50 ml Dimethylformamid, versetzt mit 25 ml 37-proz. Formaldehydlösung und 12,5 ml 88-proz. Ameisensäure und erwärmt 5 Stunden auf 100°. Nach dem Abdestillieren der leichtflüchtigen Anteile bei 70° im Wasserstrahlvakuum versetzt man den Rückstand mit 3N-Natronlauge und extrahiert zweimal mit je 500 ml Aether. Die organische Phase wird mit Wasser gewaschen, über Magnesiumsulfat getrocknet und eingedampft. Das erhaltene gelbe Oel kristallisiert beim Versetzen mit Isopropyläther. Man erhält rac-(1S*)-cis-2-/(R*)-3,4-Dichlor-α-[(dimethylamino)·methyl] benzyl/cyclohexanol in Form farbloser Kristalle vom Smp. 121-122°. Das auf übliche Weise bereitete rac-(1S*)-cis-2-/(R*)-3,4-Dichlor-α-[(dimethylamino) methyl] benzyl/cyclohexanol-Hydrochlorid wird aus Methylenchlorid/Isopropyläther umkristallisiert und schmilzt bei 259-260° (Zersetzung).

Beispiel 6

a) Zu einer Lösung von 90,3 g (0,54 Mol) N-(1-Cyclohexen-1-yl) morpholin in 500 ml Methylenchlorid unter Stickstoff wird unter Rühren und Eiskühlung eine Lösung von 89,8 g (0,54 Mol) 4-Fluor-ω-nitrostyrol in 300 ml Methylenchlorid innert etwa 85 Minuten so zugetropft, dass die Temperatur 5° nicht übersteigt. Man rührt dann während 4 Stunden bei Raumtemperatur, versetzt unter intensivem Rühren rasch mit 295 ml 2N-Salzsäure und rührt während 30 Minuten. Die wässrige Phase wird abgetrennt und noch zweimal mit je 500 ml Methylenchlorid extrahiert ; die organische Phase wird mit Wasser gewaschen, über Magnesiumsulfat getrocknet und eingedampft. Der erhaltene ölige Rückstand wird in Isopropyläther aufgenommen, und die Lösung wird über Nacht im Eisschrank stehen gelassen. Man erhält rac-(2S*)-2-[(R*)-4-Fluor-α-(nitromethyl) benzyl] cyclohexanon in Form farbloser Kristalle vom Smp. 84-85°.

b) Zu einer Suspension von 38 g (1 Mol) Lithiumaluminiumhydrid in 500 ml trockenem Tetrahydrofuran unter Argon wird unter Rühren eine Lösung von 105 g (0,4 Mol) rac-(2S*)-2-[(R*)-4-Fluor-α-(nitromethyl) benzyl] cyclohexanon in 1 l trockenem Tetrahydrofuran so zugetropft, dass die Temperatur 50° nicht übersteigt. Das Reaktionsgemisch wird während 15 Stunden bei 50° gerührt, abgekühlt und dann mit Tetrahydrofuran/Wasser (1 : 1) behandelt. Der ausgefallene Niederschlag wird unter Waschen mit Methylenchlorid abgesaugt, und das Filtrat wird abdestilliert. Man versetzt das erhaltene Oel mit 3N-Salzsäure, extrahiert zweimal mit je 500 ml Aether, stellt die wässrige Phase mit Ammoniak alkalisch und extrahiert mit Aether. Die organische Phase wird mit Wasser gewaschen, über Magnesiumsulfat getrocknet und eingedampft. Das erhaltene Oel wird in 250 ml Isopropyläther gelöst, worauf man zum Kristallisieren über Nacht im Eischrank stehen lässt. Man erhält rac-(1S*)-cis-2-[(R*)-α-(Aminomethyl)-4-fluorbenzyl] cyclohexanol in Form farbloser Kristalle vom Smp. 75-76°. Das auf übliche Weise bereitete rac-(1S*)-cis-2-[(R*)-α-(Aminomethyl)-4-fluorbenzyl] cyclohexanol-Hydrochlorid wird aus Aethanol/Aether umkristallisiert ; man erhält farblose Kristalle vom Smp. 233-234°.

c) 7,1 g (30 mMol) rac-(1S*)-cis-2-[(R*)-α-(Aminomethyl)-4-fluorbenzyl] cyclohexanol werden in 60 ml Dimethylformamid gelöst, worauf man mit 30 ml 35-proz. Formaldehydlösung und 15 ml 88-proz. Ameisensäure versetzt und während 3 Stunden bei 100° rührt. Nach dem Abdestillieren der leichtflüchtigen Anteile bei 75° im Wasserstrahlvakuum versetzt man den Rückstand mit 3N-Natronlauge und extrahiert mit Aether. Die organische Phase wird mit Wasser gewaschen, über Magnesiumsulfat getrocknet und eingedampft. Das erhaltene Oel kristallisiert nach Behandeln mit Hexan, und man erhält rac-(1S*)-cis-2-/(R*)-4-Fluor-α-[(dimethylamino)-methyl] benzyl/cyclohexanol in Form farbloser Kristalle vom Smp. 72-73°. Das auf übliche Weise bereitete Hydrochlorid wird aus Aethanol/Aether umkristallisiert, wobei man farblose Kristalle vom Smp. 240-241° erhält.

Beispiel 7

a) Zu einer Lösung von 16,7 g (0,1 Mol) N-(1-Cyclohexen-1-yl) morpholin in 50 ml Acetonitril unter Stickstoff wird unter Rühren bei — 20° eine Lösung von 17,9 g (0,1 Mol) 3-Methoxy-ω-nitrostyrol in 80 ml Acetonitril zugetropft, worauf man noch während 3 Stunden bei Raumtemperatur rührt. Nach Abdestillieren des Lösungsmittels versetzt man mit 360 ml 10-proz. Salzsäure, rührt während 2 Stunden bei Raumtemperatur, saugt den ausgefallenen Niederschlag ab und nimmt diesen in Methylenchlorid auf. Die organische Phase wird mit Wasser gewaschen, über Magnesiumsulfat getrocknet und eingedampft. Der kristalline Rückstand wird aus Methylenchlorid/Hexan umkristallisiert, wobei man rac-(2S*)-2-[(R*)-3-Methoxy-α-(nitromethyl) benzyl] cyclohexanon in Form farbloser Kristalle vom Smp. 125-126° erhält.

b) Zu einer Suspension von 14,2 g (0,38 Mol) Lithiumaluminiumhydrid in 400 ml trockenem Tetrahydrofuran unter Argon wird eine Lösung von 41,6 g (0,15 Mol) rac-(2S*)-2-[(R*)-3-Methoxy-α-(nitromethyl) benzyl] cyclohexanon in 400 ml trockenem Tetrahydrofuran zugetropft. Man erhitzt dann während 4 Stunden unter Rückfluss zum Sieden, behandelt das Reaktionsgemisch nach dem Abkühlen zuerst mit 50 ml Aethanol und anschliessend mit Tetrahydrofuran/Wasser (1 : 1), versetzt mit 80 g Kaliumcarbonat und rührt während 30 Minuten. Man saugt den ausgefallenen Niederschlag unter Waschen mit Methylenchlorid ab und dampft das Filtrat ein. Man versetzt den Rückstand mit 3N-Salzsäure und extrahiert zweimal mit je 250 ml Aether. Die wässrige Phase wird mit 3N-Natronlauge alkalisch gestellt und zweimal mit je 500 ml Aether extrahiert. Die organische Phase wird zweimal mit je 50 ml Kochsalzlösung gewaschen, über Magnesiumsulfat getrocknet und eingedampft. Das als gelbliches Oel erhaltene Rohprodukt wird in das Oxalat übergeführt. das aus Aceton umkristallisiert wird. Das erhaltene rac-(1S*)-cis-2-[(R*)-α-(Aminomethyl)-3-methoxybenzyl] cyclohexanol-Oxalat (2 : 1) schmilzt bei 210-212°.

c) Man löst 2,5 g (10 mMol) rac-(1S*)-cis-2-[(R*)-α-(Aminomethyl)-3-methoxybenzyl] cyclohexanol (aus Oxalat erhalten) in 20 ml Dimethylformamid, versetzt mit 2 ml 35-proz. Formaldehydlösung und 1 ml 88 proz. Ameisensäure und erwärmt während 2 Stunden auf 100°. Nach dem Abkühlen wird mit 3N-Natronlauge versetzt und dreimal mit je 50 ml Aether extrahiert. Die ätherische Lösung wird mit 1N-Salzsäure ausgezogen, und die wässrige Phase wird durch Zugabe von 3N-Natronlauge alkalisch gestellt. Man extrahiert dreimal mit je 100 ml Methylenchlorid, wäscht die organische Phase mit Kochsalzlösung, trocknet sie über Magnesiumsulfat und dampft sie ein. Das erhaltene Oel wird an der 20-fachen Menge Aluminiumoxid (Aktivitätsstufe II, neutral) unter Eluieren mit Toluol chromatographiert. Man erhält rac-(1S*)-cis-2-/(R*)-3-Methoxy-α-[(dimethylamino) methyl] benzyl/cyclohexanol in Form eines farblosen Oels, das auf die übliche Weise in das rac-(1S*)-cis-2-/(R*)-3-Methoxy-α-[(dimethylamino) methyl] benzyl/cyclohexanol-Hydrochlorid übergeführt wird. Durch Umkristallisieren aus Aethanol/Aether erhält man farblose Kristalle vom Smp. 188-189°.

Beispiel 8

a) Zu einer Lösung von 56,8 g (0,34 Mol) N-(1-Cyclohexen-1-yl) morpholin in 160 ml Methylenchlorid. unter Stickstoff wird innert etwa 2 Stunden eine Lösung von 87,3 g (0,34 Mol) 3-Benzyloxy-ω-nitrostyrol in 320 ml Methylenchlorid unter Rühren und Kühlen so zugetropft, dass die Temperatur 0° nicht übersteigt. Man rührt noch während 2 Stunden bei 0 bis 5°, versetzt unter intensivem Rühren auf einmal mit 445 ml 1N-Salzsäure und rührt noch während 1 Stunde. Die wässrige Phase wird abgetrennt und zweimal mit je 500 ml Methylenchlorid extrahiert ; die organische Phase wird dreimal mit je 250 ml Wasser gewaschen, über Magnesiumsulfat getrocknet und eingedampft. Der erhaltene ölige Rückstand wird in 200 ml

Methylenchlorid gelöst, worauf man mit 1 l Isopropyläther versetzt und über Nacht im Eisschrank stehenlässt. Man erhält rac-(2S*)-2-[(R*)-3-(Benzyloxy)-α-(nitromethyl) benzyl] cyclohexanon in Form farbloser Kristalle vom Smp. 119-120°.

b) Zu einer Suspension von 28,6 g (0,75 Mol) Lithiumaluminiumhydrid in 500 ml trockenem Tetrahydrofuran unter Argon wird eine Lösung von 106,6 g (0,3 Mol) rac-(2S*)-2-[(R*)-3-(Benzyloxy)-α-(nitromethyl) benzyl] cyclohexanon in 500 ml trockenem Tetrahydrofuran unter Rühren innert etwa 3 Stunden so zugetropft, dass die Temperatur 55° nicht übersteigt. Man rührt über Nacht bei 50°, behandelt das Reaktionsgemisch nach dem Abkühlen mit 40 ml Aethanol und anschliessend mit 200 ml Tetrahydrofuran/Wasser (1 : 1), saugt den ausgefallenen Niederschlag unter Waschen mit Methylenchlorid ab und dampft das Filtrat ein. Der Rückstand wird aus Methylenchlorid/Isopropyläther kristallisiert, wobei man rac-(1S*)-cis-2-[(R*)-α-(Aminomethyl)-3-(benzyloxy) benzyl] cyclohexanol in Form farbloser Kristalle vom Smp. 139-141° erhält.

c) Eine Lösung von 28,4 g (87,3 mMol) rac-(1S*)-cis-2-[(R*)-α-(Aminomethyl-3-(benzyloxy) benzyl] cyclohexanol in 175 ml Dimethylformamid wird mit 17,7 ml 37-proz. Formaldehydlösung und 8,8 ml 88-proz. Ameisensäure versetzt und während 3 Stunden bei 100° gerührt. Nach dem Abdestillieren der leichtflüchtigen Anteile im Wasserstrahlvakuum bei 75° wird der Ruckstand mit 3N-Natronlauge versetzt, worauf man mit Aether extrahiert. Die organische Phase wird mit Wasser gewaschen, über Magnesiumsulfat getrocknet und eingedampft. Das erhaltene Oel wird an der 10-fachen Menge Aluminiumoxid (Aktivitätsstufe II, neutral) unter Eluieren mit Toluol chromatographiert. Das dabei erhaltene Oel wird in Hexan gelöst, worauf man zum Kristallisieren über Nacht im Eisschrank stehen lässt. Man erhält rac-(1S*)-cis-2-/(R*)-3-(Benzyloxy)-α-[(dimethylamino) methyl] benzyl/cyclohexanol in Form farbloser Kristalle vom Smp. 85-86°.

d) Eine Lösung von 5,0 g (14,3 mMol) rac-(1S*)-cis-2-/(R*)-3-(Benzyloxy)-α-(dimethylamino) methyl] benzyl/cyclohexanol in 50 ml Aethanol wird mit 0,5 g 5-proz. Palladium/Kohle versetzt und bei Raumtemperatur hydriert. Nach Abtrennen des Katalysators wird das Lösungsmittel abdestilliert, und der kristalline Rückstand wird aus Methylenchlorid/Isopropyläther umkristallisiert. Man erhält rac-(1S*)-cis-2-/(R*)-α-[(Dimethylamino) methyl]-3-hydroxybenzyl/cyclohexanol in Form farbloser Kristalle vom Smp. 171-173°. Das daraus auf die übliche Weise bereitete rac-(1S*)-cis-2-/(R*)-α-[(Dimethylamino) methyl]-3-hydroxylbenzyl/cyclohexanol-Hydrochlorid wird aus Aethanol/Essigester umkristallisiert ; man erhält farblose Kristalle vom Smp. 191-193°.

## Beispiel 9

a) Zu einer Suspension von 47,5 g (1,25 Mol) Lithiumaluminiumhydrid in 1 l trockenem Tetrahydrofuran wird eine Lösung von 142 g (0,51 Mol) (2S*)-2-[(R*)-4-Methoxy-α-nitromethyl) benzyl] cyclohexanon in 1 200 ml trockenem Tetrahydrofuran unter Rühren so zugetropft, dass die Temperatur 50° nicht übersteigt. Anschliessend rührt man über Nacht bei 50°, kühlt das Reaktionsgemisch auf Raumtemperatur ab, versetzt mit Tetrahydrofuran/Wasser (1 : 1), saugt den ausgefallenen Niederschlag unter Waschen mit Methylenchlorid ab und dampft das Filtrat ein. Das erhaltene gelbe Oel wird in Isopropyläther gelöst, worauf man mit Hexan bis zur Trübung versetzt und über Nacht im Eisschrank stehen lässt. Man erhält rac-(1S*)-cis-2-[(R*)-α-(Aminomethyl)-4-methoxybenzyl] cyclohexanol vom Smp. 79-81°. Das auf übliche Weise bereitete rac-(1S*)-cis-2-[(R*)-α-(Aminomethyl)-4-methoxybenzyl] cyclohexanol-Hydrochlorid wird aus Aethanol/Aether umkristallisiert und schmilzt dann bei 216-218°.

b) Man rührt eine Lösung von 7,5 g (30 mMol) rac-(1S*)-cis-2-[(R*)-α-(Aminomethyl)-4-methoxybenzyl] cyclohexanol in einem Gemisch aus 60 ml Dimethylformamid, 30 ml 35-proz. Formaldehydlösung und 15 ml 88-proz. Ameisensäure während 2 Stunden bei 100°, destilliert die leichtflüchtigen Anteile bei 70° im Wasserstrahlvakuum ab, versetzt den Rückstand mit 3N-Natronlauge und extrahiert mit Aether. Die organische Phase wird mit Wasser gewaschen, über Magnesiumsulfat getrocknet und eingedampft. Das erhaltene Oel wird an der 10-fachen Menge Aluminiumoxid (Aktivitätsstufe II, neutral) unter Eluieren mit 500 ml Toluol/Aether (1 : 1) chromatographiert, wobei man ein farbloses Oel erhält, das beim Behandeln mit Hexan kristallisiert. Man erhält rac-(1S*)-cis-2-/(R*)-4-Methoxy-α-[(dimethylamino) methyl] benzyl/cyclohexanol in Form farbloser Kristalle vom Smp. 69-70°. Das auf übliche Weise bereitete rac-(1S*)-cis-2-/(R*)-4-Methoxy-α-[(dimethylamino) methyl] benzyl/cyclohexanol-Hydrochlorid wird aus Aethanol/Aether umkristallisiert ; man erhält farblose Kristalle vom Smp. 199-200°.

## Beispiel 10

a) Zu einer Lösung von 115 g (1 Mol) N-(1-Cyclohexen-1-yl) morpholin in 250 ml Methylenchlorid unter Stickstoff wird unter Rühren und Kühlen eine Lösung von 134,9 g (0,53 Mol) 4-Benzyloxy-ω-nitrostyrol in 500 ml Methylenchlorid innert etwa 2 Stunden so zugetropft, dass die Temperatur 5° nicht übersteigt. Man rührt dann während 3 Stunden bei 5°, versetzt unter intensivem Rühren rasch mit 580 ml 1N-Salzsäure und rührt noch während 1 Stunde. Die wässrige Phase wird abgetrennt und zweimal mit je

500 ml Methylenchlorid extrahiert ; die organische Phase wird dreimal mit je 200 ml Wasser gewaschen, über Magnesiumsulfat getrocknet und auf etwa 300 ml eingeengt. Nach Versetzen mit 500 ml Isopropyläther lässt man über Nacht im Eisschrank stehen. Man erhält rac-(2S*)-2-[(R*)-4-(Benzyloxy)-α-(nitromethyl) benzyl]-cyclohexanon in Form farbloser Kristalle vom Smp. 143-144°.

b) Zu einer Suspension von 28,6 g (0,75 Mol) Lithium-aluminiumhydrid in 500 ml trockenem Tetrahydrofuran unter Argon wird unter Rühren eine Lösung von 106,6 g (0,33 Mol) rac-(2S*)-2-[(R*)-4-(Benzyloxy)-α-(nitromethyl) benzyl]-cyclohexanon in 500 ml trockenem Tetrahydrofuran so zugetropft, dass die Temperatur 50° nicht übersteigt. Man rührt das Reaktionsgemisch über Nacht bei 50° und behandelt es nach dem Abkühlen auf Raumtemperatur mit 30 ml Aethanol und anschliessend mit 10 ml Tetrahydrofuran/Wasser (1 : 1). Der ausgefallene Niederschlag wird unter Waschen mit Methylenchlorid abgesaugt, und das Filtrat wird eingedampft. Das erhaltene kristalline Produkt wird aus Methylenchlorid/Isopropyläther umkristallisiert. Man erhält rac-(1S*)-cis-2-[(R*)-α-(Aminomethyl)-4-(benzyloxy) benzyl] cyclohexanol in Form farbloser Kristalle vom Smp. 160-161°.

c) Eine Lösung von 31,4 g (96,5 mMol) rac-(1S*)-cis-2-[(R*)-α-(Aminomethyl)-4-(benzyloxy) benzyl] cyclohexanol in 200 ml Dimethylformamid wird mit 19,3 ml 37-proz. Formaldehydlösung und 9,7 ml 88-proz. Ameisensäure versetzt und während 3,5 Stunden bei 100° gehalten. Nach dem Abdestillieren der leichtflüchtigen Anteile im Wasserstrahlvakuum bei 70° versetzt man den Rückstand mit 3N-Natronlauge und extrahiert mit Methylenchlorid. Die organische Phase wird mit Wasser gewaschen, über Magnesiumsulfat getrocknet und eingedampft. Das erhaltene Oel nimmt man in wenig Methylenchlorid auf, versetzt mit Isopropyläther und lässt über Nacht im Eisschrank stehen. Man erhält rac-(1S*)-cis-2-[(R*)-4-(Benzyloxy-α-[(dimethylamino) methyl] benzyl/cyclohexanol vom Smp. 123-124°.

d) Eine Lösung von 19,3 g (54,6 mMol) rac-(1S*)-cis-2-/(R*)-4-(Benzyloxy)-α-[(dimethylamino) methyl] benzyl/cyclohexanol in 200 ml Aethanol wird nach Zugabe von 1,9 g 5-proz. Palladium/Kohle bei Raumtemperatur hydriert. Nach Abtrennen des Katalysators wird das Lösungsmittel abdestilliert, und der erhaltene kristalline Rückstand wird aus Aethanol umkristallisiert. Man erhält rac-(1S*)-cis-2-/(R*)-α-[(Dimethylamino) methyl]-4-hydroxybenzyl/cyclohexanol in Form farbloser Kristalle vom Smp. 211-212°. Das auf übliche Weise bereitete Hydrochlorid wird aus Aethanol/Essigester umkristallisiert, wobei man farblose Kristalle vom Smp. 201-202° erhält.

## Beispiel 11

a) Zu einer Lösung von 29,9 g (159 mMol) N-(1-Cyclohexen-1-yl) morpholin in 60 ml Methylenchlorid unter Stickstoff wird unter Kühlen und Rühren eine Lösung von 29,1 g (134 mMol) 4-Trifluormethyl-ω-nitrostyrol in 175 ml Methylenchlorid innert etwa 45 Minuten so zugetropft, dass die Temperatur 5° nicht übersteigt. Anschliessend wird während 2 Stunden gerührt. Unter intensivem Rühren werden dann, nach Zugabe von Eis, 170 ml 1N-Salzsäure rasch zugegeben, worauf noch während 1 Stunde gerührt wird. Die wässrige Phase wird abgetrennt und zweimal mit je 200 ml Methylenchlorid extrahiert, und die organische Phase wird dreimal mit je 150 ml Wasser gewaschen, über Magnesiumsulfat getrocknet und eingedampft. Der Rückstand wird aus Isopropyläther umkristallisiert. Man erhält rac-(2S*)-2-[(R*)-α-(Nitromethy)-4-(trifluormethyl) benzyl] cyclohexanon in Form farbloser Kristalle vom Smp. 107-108°.

b) Zu einer Suspension von 4,42 g (116,5 mMol) Lithium-aluminiumhydrid in 100 ml trockenem Tetrahydrofuran unter Argon wird unter Rühren eine Lösung von 15 g (47,7 mMol) rac-(2S*)-2-[(R*)-α-(Nitromethyl)-4-(trifluormethyl) benzyl]-cyclohexanon in 110 ml trockenem Tetrahydrofuran so zugetropft, dass die Temperatur 50° nicht übersteigt. Man rührt das Reaktionsgemisch über Nacht bei 50°, kühlt ab, behandelt mit Aethanol und anschliessend mit Tetrahydrofuran/Wasser (1 : 1), saugt den ausgefallenen Niederschlag unter Waschen mit Methylenchlorid ab und dampft das Filtrat ein. Der ölige Rückstand kristallisiert beim Behandeln mit Isopropyläther. Man erhält rac-(1S*)-cis-2-[(R*)-α-(Aminomethyl)-4-trifluormethyl) benzyl] cyclohexanol in Form schwach rötlicher Kristalle vom Smp. 103-105°.

c) Eine Lösung von 9,2 g (32 mMol) rac-(1S*)-cis-2-[(R*)-α-(Aminomethyl)-4-trifluormethyl) benzyl] cyclohexanol in 65 ml Dimethylformamid wird mit 6,5 ml 35-proz. Formaldehydlösung und 3,5 ml 88-proz. Ameisensäure während 2 Stunden bei 100° gerührt. Nach dem Abdestillieren der leichtflüchtigen Anteile im Wasserstrahlvakuum bei 75° wird der Rückstand mit 3N-Natronlauge versetzt, worauf man zweimal mit je 250 ml Methylenchlorid extrahiert. Die organische Phase wird mit 50 ml Wasser gewaschen, über Magnesiumsulfat getrocknet und eingedampft. Das erhaltene gelbe Oel wird an der 30-fachen Menge Aluminiumoxid (Aktivitätsstufe II, neutral) unter Eluieren mit Toluol chromatographiert. Aus dem resultierenden kristallinen Rückstand erhält man nach Behandeln mit Hexan rac-(1S*)-cis-2-/(R*)-α-[(Dimethylamino)-methyl]-4-(trifluormethyl) benzyl/cyclohexanol vom Smp. 105-106°. Das daraus auf übliche Weise bereitete Hydrochlorid wird aus Alkohol/Aether umkristallisiert ; man erhält farblose Kristalle vom Smp. 263°.

Beispiel 12

a) Zu einer Suspension von 15,4 g (407 mMol) Lithium-aluminiumhydrid in 350 ml trockenem Tetrahydrofuran unter Argon wird unter Rühren eine Lösung von 50,5 g (163 mMol) rac-(2S*)-2-[(R*)-3,4-Dimethoxy-α-(nitromethyl) benzyl] cyclohexanon in 500 ml trockenem Tetrahydrofuran so zugetropft, dass die Temperatur 50° nicht übersteigt. Man rührt über Nacht bei 50° und behandelt das Reaktionsgemisch nach dem Abkühlen zuerst mit Aethanol und anschliessend mit Tetrahydrofuran/Wasser (1 : 1). Der ausgefallene Niederschlag wird unter Waschen mit Methylenchlorid abgesaugt und das Filtrat wird eingedampft. Das erhaltene, ölige Rohprodukt wird in das Hydrochlorid übergeführt, das aus Alkohol umkristallisiert wird. Man erhält rac-(1S*)-cis-2-[(R*)-α-(Aminomethyl)-3,4-dimethoxybenzyl] cyclohexanol-Hydrochlorid vom Smp. 216-218°.

b) Eine Lösung von 11,2 g (40,1 mMol) rac-(1S*)-cis-2-[(R*)-α-(Aminomethyl)-3,4-dimethoxybenzyl] cyclohexanol (aus dem Hydrochlorid erhalten) in 80 ml Dimethylformamid wird mit 8 ml 37-proz. Formaldehydlösung und 4 ml 88-proz. Ameisensäure versetzt und während 2,5 Stunden bei 100° gerührt. Nach Abdestillieren der leichtflüchtigen Anteile im Wasser strahlvakuum bei 70° wird der Rückstand in Aether aufgenommen. Man extrahiert mit 3N-Salzsäure, stellt die saure Phase durch Zugabe von konz. Ammoniak alkalisch und extrahiert dreimal mit je 100 ml Methylenchlorid. Die organische Phase wird mit Wasser gewaschen, über Magnesiumsulfat getrocknet und eingedampft. Das erhaltene Oel wird an der 10-fachen Menge Aluminiumoxid (Aktivitätsstufe II, neutral) unter Eluieren mit Toluol chromatographiert. Man erhält das Produkt als farbloses Oel, das in das Hydrochlorid übergeführt wird. Nach Umkristallisieren aus Aethanol/Aether erhält man rac-(1S*)-cis-2-/(R*)-α-[(Dimethylamino) methyl]-3,4-dimethoxybenzyl/cyclohexanol-Hydrochlorid in Form farbloser Kristalle vom Smp. 188-190°.

Beispiel 13

a) Zu einer Lösung von 28,2 g (100 mMol) rac-(2S*)-2-[(R*)-4-Chlor-α-(nitromethyl) benzyl] cyclohexanon in 200 ml Methanol wird unter Kühlen eine Lösung von 6,8 g (121 mMol) Kaliumhydroxid in 40 ml Wasser innert etwa 30 Minuten so zugetropft, dass die Temperatur 5° nicht übersteigt. Anschliessend wird eine eiskalte Lösung von 2,85 g (75 mMol) Natriumborhydrid in 20 ml Wasser zugetropft, worauf man während 3 Stunden bei 0° rührt. Nach Versetzen mit 12 ml Essigsäure wird das Lösungsmittel abdestilliert. Der erhaltene Rückstand wird mit Wasser versetzt, worauf man zweimal mit je 200 ml Aether extrahiert. Die organische Phase wird mit Wasser gewaschen, über Magnesiumsulfat getrocknet und eingedampft. Man nimmt das erhaltene Oel in Aether/Pentan auf, lässt im Eisschrank stehen und kristallisiert das erhaltene kristalline Produkt aus Aether/Hexan um. Man erhält rac-(1S*)-cis-2-[(R*)-4-Chlor-α-(nitromethyl) benzyl] cyclohexanol vom Smp. 82-84°.

b) 9,1 g (32,1 mMol) rac-(1S*)-cis-2-[(R*)-4-Chlor-α-(nitromethyl) benzyl] cyclohexanol, 16,8 g (64,2 mMol) Triphenylphosphin und 10,8 g (64,2 mMol) p-Nitrobenzoesäure werden in 180 ml Tetrahydrofuran gelöst. Zu dieser Lösung wird innert 30 Minuten eine Lösung von 11,3 g (64,2 mMol) Azodicarbonsäurediäthylester in 80 ml Tetrahydrofuran zugetropft, und anschliessend wird während 5 Stunden bei Raumtemperatur gerührt. Nach Abdestillieren des Lösungsmittels wird der erhaltene Rückstand an der 30-fachen Menge Kieselgel unter Eluieren mit Toluol/Hexan (1 : 2) chromatographiert. Durch Umkristallisieren des erhaltenen Produktes aus Methylenchlorid/Hexan erhält man rac-(1R*)-trans-2-[(R*)-4-Chlor-α-(nitromethyl) benzyl] cyclohexyl-p-nitrobenzoat in Form farbloser Kristalle vom Smp. 146-148°.

c) Zu einer Lösung von 3,5 g (8,09 mMol) rac-(1R*)-trans-2-[(R*)-4-Chlor-α-(nitromethyl) benzyl] cyclohexyl-p-nitrobenzoat in 80 ml Aethanol wird eine Lösung von 490 ml (12,1 mMol) Natriumhydroxid in 40 ml Aethanol zugetropft. Man rührt während 16 Stunden bei Raumtemperatur, versetzt mit 1 ml Essigsäure und destilliert das Lösungsmittel im Wasserstrahlvakuum ab. Der Rückstand wird mit Wasser versetzt, worauf man zweimal mit je 100 ml Aether extrahiert.

Die organische Phase wird mit Wasser gewaschen, über Magnesiumsulfat getrocknet und eingedampft. Man chromatographiert den Rückstand an der 30-fachen Menge Kieselgel und erhält rac-(1R*)-trans-2-[(R*)-4-Chlor-α-(nitromethyl) benzyl] cyclohexanol in Form eines gelblichen Oels.

d) Zu einer Suspension von 1 g Lithiumaluminiumhydrid in 60 ml trockenem Tetrahydrofuran unter Argon wird unter Rühren eine Lösung von 1,9 g (6,7 mMol) rac-(1R*)-trans-2-[(R*)-4-Chlor-α-(nitromethyl) benzyl] cyclohexanol in 30 ml trockenem Tetrahydrofuran zugetropft, worauf man noch über Nacht bei 50° rührt. Nach dem Abkühlen behandelt man das Reaktions-gemisch mit Tetrahydrofuran/Wasser (1 : 1), saugt den Niederschlag ab, dampft das Filtrat ein, versetzt den Rückstand mit Wasser und extrahiert mit Aether. Das erhaltene rac-(1R*)-trans-2-[(R*)-α-(Aminomethyl)-4-chlorbenzyl] cyclohexanol ist ein farbloses Oel und wird direkt weiter verarbeitet.

e) Eine Lösung von 1,55 g (6,1 mMol) rac-(1R*)-trans-2-[(R*)-α-(Aminomethyl)-4-chlorbenzyl] cyclohexanol in 30 ml Dimethylformamid wird mit 1,5 ml 35-proz. Formaldehydlösung und 0,8 ml 88-proz.

Ameisensäure versetzt und während 2 Stunden bei 100° gehalten. Nach dem Abkühlen wird das Lösungsmittel bei 75° im Wasserstrahlvakuum abdestilliert ; der Rückstand wird mit Wasser versetzt, worauf man mit Aether extrahiert. Die ätherische Lösung wird mit 1N-Salzsäure ausgezogen. die saure Phase wird mit konz. Ammoniak alkalisch gestellt und mit Methylenchlorid extrahiert. Die organische Phase wird mit Wasser gewaschen, über Magnesiumsulfat getrocknet und eingedampft. Man erhält rac-(1R*)-trans-2-/(R*)-4-Chlor-α-[(dimethylamino) methyl] benzyl/cyclohexanol vom Smp. 89-90°. Das daraus auf übliche Weise bereitete rac-(1R*)-trans-2/(R*)-4-Chlor-α-[(dimethylamino)methyl] benzyl/cyclohexanol-Hydrochlorid wird aus Acetonitril/Aether umkristallisiert und schmilzt bei 171-173°.

## Beispiel 14

Die in Beispiel 1 b) nach Abtrennen des kristallinen rac-(1S*)-cis-2-[(R*)-α-(Aminomethyl)-4-chlorbenzyl] cyclohexanols anfallende Mutterlauge wird eingedampft. Der Rückstand (54 g), der etwa 20 % rac-(1R*)-trans-2-[(R*)-α-(Amino-methyl)-4-chlorbenzyl] cyclohexanol enthält, wird in 250 ml Dimethylformamid gelöst. Man versetzt mit 49,5 ml 35-proz. Formaldehydlösung und 25 ml 88-proz. Ameisensäure und hält während 3 Stunden bei 100°. Die leichtflüchtigen Anteile werden im Wasserstrahlvakuum bei 75° abdestilliert, worauf man den Rückstand mit 3N-Natronlauge versetzt und mit Aether extrahiert. Die organische Phase wird mit Wasser gewaschen, über Magnesiumsulfat getrocknet und eingedampft. Das erhaltene Oel wird an der 30-fachen Menge Aluminiumoxid (Aktivitätsstufe II, neutral) unter Eluieren mit Toluol chromatographiert. Man erhält rac-(1R*)-trans-2-/(R*)-4-Chlor-α-[(dimethylamino) methyl] benzyl/cyclohexanol als farblose Kristalle vom Smp. 89-90°.

## Beispiel 15

a) Zu einer Lösung von 50,0 g (180 mMol) rac-(2S*)-2-[(R*)-4-Chlor-α-(nitromethyl) benzyl] cyclohexanon in 500 ml Methanol wird unter Argon eine Lösung von 12,0 g (214 mMol) Kaliumhydroxid in 1 l Methanol rasch zugegeben. Man rührt während 1 Stunde auf 0° ab, versetzt mit 12,2 ml (214 mMol) Essigsäure und destilliert das Lösungsmittel im Wasserstrahlvakuum ab. Man versetzt den Rückstand mit Wasser, extrahiert zweimal mit je 500 ml Aether, wäscht die ätherische Phase zweimal mit je 50 ml Wasser, trocknet sie über Magnesiumsulfat und dampft sie ein. Das erhaltene Oel enthält etwa 41 % des zum eingesetzten Ausgangsmaterial isomeren Ketons. Zur Reinigung wird an 1 kg Kieselgel unter Eluieren mit Toluol/Essigester (30 : 1) chromatographiert. Aus dem erhaltenen Material erhält man nach Umkristallisation aus Methanol rac-(2S*)-2-[(S*)-4-Chlor-α-(nitromethyl)-benzyl] cyclohexanon in Form farbloser Kristalle vom Smp. 87-88°.

b) Zu einer Suspension von 1,7 g (44,8 mMol) Lithiumaluminiumhydrid in 50 ml trockenem Tetrahydrofuran unter Argon wird unter Rühren eine Lösung von 5,0 g (17,7 mMol) rac-(2S*)-2-[(S*)-4-Chlor-α-(nitromethyl) benzyl]-cyclohexanon in 50 ml trockenem Tetrahydrofuran zugetropft. Man rührt über Nacht bei 50° und behandelt das Reaktionsgemisch nach dem Abkühlen mit 20 ml Aethanol und anschliessend mit 30 ml Tetrahydrofuran/Wasser (1 : 1). Man versetzt mit 100 ml Methylenchlorid, saugt den Niederschlag unter Waschen mit Methylenchlorid ab und dampft das Filtrat ein. Nach Versetzen des Rückstandes mit Wasser extrahiert man mit Aether, schüttelt die ätherische Lösung mit 3N-Salzsäurelösung aus, versetzt die saure Phase mit 3N-Natronlauge und extrahiert mit Methylenchlorid. Die organische Phase wird mit Wasser gewaschen, über Magnesiumsulfat getrocknet und eingedampft. Das resultierende Oel enthält etwa 74 % des äquatorialen und etwa 19 % des axialen Alkohols. Durch Chromatographieren an Kieselgel mit Methylenchlorid/Methanol (10 : 1) wird zuerst der axiale Alkohol rac-(1S*)-cis-2-[(S*)-α-(Aminomethyl)-4-chlorbenzyl] cyclohexanol und dann der äquatoriale Alkohol rac-(1R*)-trans-2-[(S*)-α-(Aminomethyl)-4-chlorbenzyl]-cyclohexanol isoliert. Durch Umkristallisieren des axialen Alkohols aus Methanol/Aether erhält man rac-(1S*)-cis-2-[(S*)-α-(Aminomethyl)-4-chlorbenzyl] cyclohexanol in Form farbloser Kristalle vom Smp. 153-154°.

Der äquatoriale Alkohol rac-(1R*)-trans-2-[(S*)-α-(Aminomethyl)-4-chlorbenzyl] cyclohexanol wird in Form farbloser Kristalle vom Smp. 138-139° erhalten.

c) Eine Lösung von 2,8 g (11,0 mMol) rac-(1S*)-cis-2-[(S*)-α-(Aminomethyl)-4-chlorbenzyl] cyclohexanol in 50 ml Dimethylformamid wird mit 2,6 ml 37-proz. Formaldehydlösung und 0,5 ml 88-proz. Ameisensäure versetzt und während 3 Stunden bei 100° gehalten. Nach dem Abkühlen wird das Lösungsmittel im Wasserstrahlvakuum bei 75° abdestilliert, und der Rückstand wird mit Wasser versetzt. Man extrahiert zweimal mit je 100 ml Aether, wäscht die organische Phase mit Wasser, trocknet sie über Magnesiumsulfat und dampft sie ein. Das erhaltene kristalline Produkt wird aus Isopropyläther/Hexan umkristallisiert. Man erhält rac-(1S*)-cis-2-/(S*)-4-Chlor-α-(dimethylamino) methyl] benzyl/cyclohexanol vom Smp. 126-128°. Das auf übliche Weise erhaltene Hydrochlorid wird aus Essigester umkristallisiert ; man erhält farblose Kristalle vom Smp. 192-193°.

Beispiel 16

a) Man versetzt auf —20° abgekühlte konz. Salpetersäure (65 %) unter Argon und unter Rühren innert etwa 1 Stunde portionenweise mit insgesamt 1,4 g (5 mMol) rac-(1S*)-cis-2-/(R*)-4-Chlor-α-[(dimethylamino) methyl] benzyl/cyclohexanol und rührt anschliessend während 2 Stunden bei —20°. Man giesst das Reaktionsgemisch auf Eiswasser, stellt durch Zugabe von 3N-Natronlauge alkalisch und extrahiert dreimal mit je 150 ml Methylenchlorid. Die organische Phase wird mit Wasser gewaschen, über Magnesiumsulfat getrocknet und eingedampft. Das erhaltene ölige Produkt wird auf übliche Weise in das Hydrochlorid übergeführt, das dann aus Alkohol/Aether umgelöst wird. Man erhält rac-(2S*)-2-/(R*)-4-Chlor-α-[(dimethylamino) methyl] benzyl/cyclohexanon-Hydrochlorid in Form farbloser Kristalle vom Smp. 175-176°.

Beispiel 17

a) Eine Lösung von 17,5 g (80 mMol) rac-(1S*)-cis-2-[(R*)-α-(Aminomethyl) benzyl] cyclohexanol in 170 ml Dimethylformamid wird mit 16 ml 35-proz. Formaldehydlösung und 8 ml 88-proz. Ameisensäure versetzt und während 3 Stunden bei 100° gehalten. Nach Abdestillieren der leichtflüchtigen Anteile bei 75° im Wasserstrahlvakuum wird der Rückstand mit 3N-Natronlauge versetzt. Man extrahiert mit Aether, wäscht die organische Phase mit Wasser, trocknet sie über Magnesiumsulfat und dampft sie ein. Das erhaltene Oel wird an der 10-fachen Menge Aluminiumoxid (Aktivitätsstufe II, neutral) unter Eluieren mit Toluol chromatographiert. Zum Kristallisieren wird das erhaltene Oel in Isopropyläther gelöst, worauf man mit Hexan versetzt und über Nacht im Eisschrank stehen lässt. Man erhält rac-(1S*)-cis-2-/(R*)-α-[(Dimethylamino) methyl] benzyl/cyclohexanol in Form farbloser Kristalle vom Smp. 95-96°. Das auf übliche Weise bereitete Hydrochlorid wird aus Aethanol/Aether umkristallisiert und schmilzt bei 212-213°.

b) Man versetzt auf —20° abgekühlte konz. Salpetersäure (65 %) unter Argon und unter Rühren innert etwa 1,5 Stunden portionenweise mit insgesamt 4,4 g (17,8 mMol) rac-(1S*)-cis-2-/(R*)-α-[(Dimethylamino)methyl] benzyl/cyclohexanol, rührt anschliessend weitere 2 Stunden bei —20°, giesst das Reaktionsgemisch auf Eis, stellt durch Zugabe von 3N-Natronlauge alkalisch und extrahiert dreimal mit je 250 ml Methylenchlorid. Die organische Phase wird mit Wasser gewaschen, über Magnesiumsulfat getrocknet und eingedampft. Das als gelbes Oel erhaltene Rohprodukt wird in das Hydrochlorid übergeführt, das aus Methylenchlorid/Aether umkristallisiert wird. Man erhält rac-(2S*)-2-/(R*)-α-[(Dimethylamino)-methyl] benzyl/cyclohexanon-Hydrochlorid in Form farbloser Kristalle vom Smp. 197-198°.

c) Zu 125 ml auf —20° abgekühlte rauchende Salpetersäure unter Argon werden unter Rühren innert etwa 1 Stunde portionenweise insgesamt 9,6 g (39,1 mMol) rac-(2S*)-2-/(R*)-α-[(Dimethylamino) methyl] benzyl/cyclohexanon eingetragen, worauf man noch während 3 Stunden bei —20° rührt. Das Reaktionsgemisch wird auf Eis gegossen. Man stellt durch Zugabe von konz. Ammoniak alkalisch und extrahiert dreimal mit je 300 ml Methylenchlorid. Die organische Phase wird mit Wasser gewaschen, über Magnesiumsulfat getrocknet und eingedampft. Das erhaltene Oel wird an der 10-fachen Menge Aluminiumoxid (Aktivitätsstufe II, neutral) unter Eluieren mit Toluol chromatographiert. Das als farbloses Oel erhaltene Rohprodukt wird auf übliche Weise in das Hydrochlorid übergeführt, das aus Aethanol/Essigester umkristallisiert wird. Man erhält rac-(2S*)-2-/(R*)-α-[(Dimethylamino) methyl]-4-nitrobenzyl/cyclohexanon-Hydrochlorid als gelbliche Kristalle vom Smp. 210-211°.

Beispiel 18

a) Die in Beispiel 10 c) nach Abtrennen des kristallinen rac-(1S*)-cis-2-/(R*)-4-(Benzyloxy)-α-[(dimethylamino)-methyl] benzyl/cyclohexanol anfallende Mutterlauge wird eingedampft. Der Rückstand enthält etwa 20 % des äquatorialen isomeren Alkohols rac-(1R*)-trans-2-/(R*)-4-[(Benzyloxy)-α-[(dimethylamino) methyl] benzyl/cyclohexanol und wird an der 30-fachen Menge Aluminiumoxid chromatographiert. Man eluiert zuerst mit Toluol den in Beispiel 10 c) beschriebenen axialen Alkohol und dann mit Aether den gewünschten äquatorialen Alkohol und dann mit Aether den gewünschten äquatorialen Alkohol. Nach Umkristallisieren aus Isopropyläther/Hexan erhält man rac-(1R*)-trans-2-/(R*)-4-(Benzyloxy)-α-[(dimethylamino) methyl] benzyl/cyclohexanol in Form farbloser Kristalle vom Smp. 98-100°.

b) Eine Lösung von 3,6 g (10,2 mMol) rac-(1R*)-trans-2-/(R*)-4-(Benzyloxy)-α-[(dimethylamino) methyl] benzyl/cyclohexanol in 50 ml Aethanol wird mit 0,4 g 5-proz. Palladium/Kohle versetzt und bei Raumtemperatur hydriert. Nach Abtrennen des Katalysators wird das Lösungsmittel abdestilliert, und der Rückstand wird mit Hexan versetzt. Man erhält rac-(1R*)-trans-2-/(R*)-α-[(Dimethylamino) methyl]-4-hydroxybenzyl/cyclohexanol in Form farbloser Kristalle vom Smp. 201-202°. Das auf übliche Weise bereitete Hydrochlorid wird aus Aethanol/Aether umkristallisiert und schmilzt bei 215-216°.

Beispiel 19

a) Zu einer Lösung von 25,7 g (142 mMol) N-(1-Cyclohepten-1-yl) morpholin in 200 ml Methylenchlorid wird unter Rühren und Kühlen eine Lösung von 20 g (109 mMol) 4-Chlor-ω-nitrostyrol in 300 ml Methylenchlorid so zugetropft, dass die Temperatur 0° nicht übersteigt. Anschliessend wird während 3 Stunden bei 0° gerührt. Unter intensivem Rühren werden dann auf einmal 200 ml 1N-Salzsäure zugegeben, worauf man noch während 1 Stunde bei 0° rührt. Die wässrige Phase wird abgetrennt und zweimal mit je 200 ml Essigester extrahiert ; die organische Phase wird mit Wasser gewaschen, über Magnesiumsulfat getrocknet und eingedampft. Das erhaltene Oel wird in Isopropyläther gelöst, worauf man mit Hexan versetzt und über Nacht im Eisschrank stehen lässt. Man erhält rac-(2S*)-2-[(R*)-4-Chlor-α-nitrobenzyl] cycloheptanon in Form farbloser Kristalle vom Smp. 77-78°.

b) Zu einer Suspension von 4,1 g (108,2 mMol) Lithium-aluminiumhydrid in 120 ml trockenem Tetrahydrofuran unter Argon wird eine Lösung von 12,8 g (43,3 mMol) rac-(2S*)-2-[(R*)-4-Chlor-α-nitrobenzyl] cycloheptanon in 160 ml trockenem Tetrahydrofuran zugetropft. Man rührt über Nacht bei 50°, kühlt ab, behandelt das Reaktionsgemisch mit Tetrahydrofuran/Wasser (1 : 1), saugt den ausgefallenen Niederschlag ab und dampft das Filtrat ein. Man versetzt den Rückstand mit Wasser und extrahiert mit Aether. Die organische Phase wird mit 3N-Salzsäure extrahiert ; die wässrige Phase wird durch Zugabe von konz. Ammoniak alkalisch gestellt und mit Methylenchlorid extrahiert. Die organische Phase wird mit Wasser gewaschen, über Magnesiumsulfat getrocknet und eingedampft. Das erhaltene Oel wird aus Methylenchlorid/Hexan umkristallisiert. Man erhält rac-(1S*)-cis-2-[(R*)-α-(Aminomethyl)-4-chlorbenzyl] cycloheptanol in Form farbloser Kristalle vom Smp. 92-93°.

c) Zu einer Lösung von 7,3 g (27,3 mMol) rac-(1S*)-cis-2-[(R*)-α-(Aminomethyl)-4-chlorbenzyl] cycloheptanol in 70 ml Dimethylformamid werden 5,5 ml 35-proz. Formaldehydlösung und 3 ml 88-proz. Ameisensäure gegeben. Man erwärmt während 2 Stunden auf 100°, destilliert die leichtflüchtigen Anteile im Wasserstrahlvakuum bei 75° ab, versetzt den Rückstand mit Wasser und konz. Ammoniak und extrahiert zweimal mit je 100 ml Methylenchlorid. Die organische Phase wird mit Wasser gewaschen, über Magnesiumsulfat getrocknet und eingedampft. Das erhaltene Oel wird an der 10-fachen Menge Aluminiumoxid unter Eluieren mit Toluol chromatographiert. Das erhaltene Oel kristallisiert beim Behandeln mit Hexan. Das erhaltene rac-(1S*)-cis-2-/(R*)-4-Chlor-α-[(dimethylamino) methyl] benzyl/cycloheptanol schmilzt bei 86-87° und das daraus auf übliche Weise erhaltene Hydrochlorid wird aus Aethanol/Aether umkristallisiert und schmilzt bei 254-255°.

Beispiel 20

a) Zu einer Suspension von 1,5 g (40,4 mMol) Lithium-aluminiumhydrid in 50 ml trockenem Tetrahydrofuran unter Argon wird eine Lösung von 4,55 g (16,1 mMol) (—)-(2S)-[(R)-4-Chlor-α-(nitromethyl) benzyl] cyclohexanon [hergestellt nach Helv. Chim. Acta 65, 1637 (1982)] in 80 ml trockenem Tetrahydrofuran unter Rühren bei Raumtemperatur zugetropft. Man rührt über Nacht bei Raumtemperatur, behandelt das Reaktionsgemisch mit 20 ml Tetrahydrofuran/Wasser (1 : 1), saugt den ausgefallenen Niederschlag unter Waschen mit Methylenchlorid ab und dampft das Filtrat ein. Der Rückstand wird in Aether gelöst, die ätherische Phase mit 1N Salzsäure extrahiert, die saure Phase mit 3N Natronlauge alkalisch gestellt und mit Methylenchlorid extrahiert. Die organische Phase wird mit Wasser gewaschen, über Magnesiumsulfat getrocknet und eingedampft. Man erhält (+)-(1S)-cis-2-[(R)-α-(Aminomethyl)-4-chlorbenzyl] cyclohexanol in Form eines gelben Oels ($[\alpha]_D^{20}$ = + 14,7°, c = 0,6 in Chloroform), das gleich weiter umgesetzt wird.

b) Man rührt eine Lösung von 2,8 g (+)-(1S)-cis-2-[(R)-α-(Aminomethyl)-4-chlorbenzyl] cyclohexanol in einem Gemisch aus 60 ml Dimethylformamid, 2,2 ml 35-proz. Formaldehyd-lösung und 1,5 ml 88-proz. Ameisensäure während 2 Stunden bei 100°, destilliert die leichtflüchtigen Anteile bei 70° im Wasserstrahlvakuum ab, versetzt den Rückstand mit 3N Natronlauge und extrahiert mit Aether. Die organische Phase wird mit Wasser gewaschen, über Magnesiumsulfat getrocknet und eingedampft. Das erhaltene Oel wird an der 20-fachen Menge Aluminiumoxid (Aktivitätsstufe II, neutral) unter Eluieren mit Toluol chromatographiert, wobei man ein farbloses Oel erhält, das beim Behandeln mit Isopropyläther/Hexan kristallisiert. Man erhält (—)-(1S)-cis-2-/(R)-4-Chlor-α-[(dimethylamino) methyl] benzyl/cyclohexanol in Form farbloser Kristalle vom Schmelzpunkt 117° ; $[\alpha]_D^{20}$ = — 15,6° (c = 1, Chloroform).

Beispiel 21

a) Eine Lösung von 26,5 g (117,8 mMol) 1-(4'-Benzyloxy-phenyl)-2-nitroäthylen in 350 ml Methylenchlorid wird auf — 75° abgekühlt und unter Rühren innert einer Stunde 25,3 g (129,6 mMol) (—)-(S)-1-(1-Cyclohexen-1-yl)-2-(methoxy-methyl) pyrrolidin [Helv. Chim. Acta 65, 1637 (1982)] zugetropft. Anschliessend wird 1 Stunde bei 0° und über Nacht bei Raumtemperatur weitergerührt. Das Reaktionsgemisch wird auf 0° abgekühlt und innert 15 Minuten mit 130 ml 1N Salzsäure versetzt und 1 Stunde weitergerührt.

Die saure Phase wird zweimal mit je 150 ml Methylenchlorid extrahiert und die organische Phase mit 100 ml 1N Salzsäure und zweimal mit je 100 ml Wasser gewaschen, über Magnesiumsulfat getrocknet und vom Lösungsmittel befreit. Das erhaltene Oel wird an der 10-fachen Menge Kieselgel mit Methylenchlorid chromatographiert. Nach Kristallisation aus Methylenchlorid/Isopropyläther erhält man (—)-(S)-2-[R)-4-(Benzyloxy)-α-(nitromethyl) benzyl] cyclohexanon in Form farbloser Kristalle vom Schmelzpunkt 168-169° ; $[\alpha]_D^{20}$ = —19,8° (c = 0,5, Chloroform).

b) Zu einer Suspension von 2,5 g (67,2 mMol) Lithium-aluminiumhydrid in 80 ml trockenem Tetrahydrofuran unter Argon wird eine Lösung von 9,5 g (—)-(S)-2-[(R)-4-(Benzyloxy)-α-(nitromethyl) benzyl] cyclohexanon in 150 ml trockenem Tetrahydrofuran unter Rühren bei Raumtemperatur zugetropft und das Gemisch während 16 Stunden bei Raumtemperatur weitergerührt. Das Reaktionsgemisch wird mit 20 ml Tetrahydrofuran/Wasser (1 : 1) versetzt, der ausgefallene Niederschlag unter Waschen mit Methylenchlorid abgesaugt und das Filtrat abgedampft. Der Rückstand wird in Aether gelöst, die ätherische Phase mit 1N Salzsäure extrahiert, die saure Phase mit 3N Natronlauge alkalisch gestellt und mit Methylenchlorid extrahiert. Die organische Phase wird mit Wasser gewaschen, über Magnesiumsulfat getrocknet und eingedampft. Man erhält (+)-(1S)-cis-2-[(R)-α-(Aminomethyl)-4-(benzyloxy) benzyl]-cyclohexanol in Form eines farblosen Oels ; $[\alpha]_D^{20}$ = + 14,7° (c = 0,7, Chloroform).

c) Man rührt eine Lösung von 7,2 g (+)-(1S)-cis-2-[(R)-α-(Aminomethyl)-4-(benzyloxy) benzyl] cyclohexanol in einem Gemisch aus 100 ml Dimethylformamid, 4,4 ml 35-proz. Formaldehydlösung und 2,5 ml 88-proz. Ameisensäure während 2 Stunden bei 100°, destilliert die leichtflüchtigen Anteile bei 70° am Wasserstrahlvakuum ab, versetzt den Rückstand mit 3N Natronlauge und extrahiert mit Aether. Die organische Phase wird mit Wasser gewaschen, über Magnesiumsulfat getrocknet und vom Lösungsmittel befreit. Das erhaltene Oel wird an der 20-fachen Menge Aluminiumoxid mit Toluol chromatographiert. Man erhält (—)-(1S)-cis-2-/(R)-4-(Benzyloxy)-α-[(dimethylamino) methyl] benzyl/cyclohexanol in Form farbloser Kristalle vom Schmelzpunkt 128-130° ; $[\alpha]_D^{20}$ = — 13° (c = 1, Aethanol).

d) 3,0 g (—)-(1S)-cis-2-/(R)-4-(Benzyloxy)-α-[(dimethylamino) methyl] benzyl/cyclohexanol werden in 100 ml Aethanol gelöst und nach Zugabe von 0,3 g 5-proz. Palladium/Kohle bei Raumtemperatur hydriert. Nach Abtrennen vom Katalysator wird das Filtrat eingeengt und der kristalline Rückstand aus Aethanol umgelöst. Man erhält (—)-(1S)-cis-2-/(R)-α-[(Dimethylamino) methyl]-4-hydroxybenzyl/cyclohexanol in Form farbloser Kristalle vom Schmelzpunkt 235-237° ; $[\alpha]_D^{20}$ = — 19,8° (c = 1, Aethanol).

Beispiel 22

a) Eine Lösung von 35,75 g (158,7 mMol) 1-(4'-Benzyloxy-phenyl)-2-nitroäthylen in 450 ml Methylenchlorid wird auf 0° abgekühlt und innert 1 Stunde unter Rühren 34,1 g (174,6 mMol) (+)-(R)-1-(1-Cyclohexen-1-yl)-2-(methoxy-methyl) pyrrolidin [Helv. Chim. Acta 65, 1637 (1982)] zugetropft. Anschliessend wird das Gemisch während 3 Stunden bei 0° und über Nacht bei Raumtemperatur weitergerührt. Das Reaktionsgemisch wird auf 0° abgekühlt und innert 15 Minuten mit 175 ml 1N Salzsäure versetzt und 1 Stunde weitergerührt. Die saure Phase wird zweimal mit je 150 ml Methylenchlorid extrahiert, die organische Phase mit 100 ml 1N Salzsäure und zweimal mit je 100 ml Wasser gewaschen, über Natriumsulfat getrocknet und vom Lösungsmittel befreit. Das erhaltene Oel wird an der 10-fachen Menge Kieselgel mit Methylenchlorid filtriert. Nach Umkristallisation aus Methylenchlorid/Isopropyläther erhält man (+)-(R)-2-[(S)-4-(Benzyloxy)-α-(nitromethyl) benzyl] cyclohexanon in Form farbloser Kristalle vom Schmelzpunkt 159-160° ; $[\alpha]_D^{20}$ = +22° (c = 0,5, Chloroform).

b) Zu einer Suspension von 2,6 g (70,7 mMol) Lithium-aluminiumhydrid in 80 ml trockenem Tetrahydrofuran unter Argon wird eine Lösung von 10,0 g (+)-(R)-2-[(S)-4-(Benzyloxy)-α-(nitromethyl) benzyl] cyclohexanon in 120 ml trockenem Tetrahydrofuran unter Rühren bei Raumtemperatur zugetropft und das Gemisch während 16 Stunden bei Raumtemperatur weitergerührt. Das Reaktionsgemisch wird mit 20 ml Tetrahydrofuran/Wasser (1 : 1) und 10 ml konz. Natronlauge versetzt, der ausgefallene Niederschlag unter Waschen mit Methylenchlorid abgesaugt und das Filtrat abgedampft. Man erhält rohes (—)-(1R)-cis-2-[(S)-α-(Aminomethyl)-4-(benzyloxy) benzyl] cyclohexanol in Form eines gelblichen Oels.

c) Man rührt eine Lösung von 9,4 g (—)-(1R)-cis-2-[(S)-α-(Aminomethyl)-4-(benzyloxy) benzyl] cyclohexanol in einem Gemisch aus 130 ml Dimethylformamid, 5,6 ml 35-proz. Formaldehydlösung und 3,3 ml 88-proz. Ameisensäure während 2 Stunden bei 100°, destilliert die leichtflüchtigen Anteile bei 70° am Wasserstrahlvakuum ab, versetzt den Rückstand mit konz. Ammoniak und extrahiert mit Methylenchlorid. Die organische Phase wird zweimal mit je 50 ml Wasser gewaschen, über Natriumsulfat getrocknet und das Lösungsmittel abgedampft. Das erhaltene Oel (7,5 g) wird an der 20-fachen Menge Aluminiumoxid mit Toluol chromatographiert. Nach Kristallisation aus Methylenchlorid/Hexan erhält man (+)-(1R)-cis-2-/(S)-4-(Benzyloxy)-α-[(dimethylamino) methyl]-benzyl/cyclohexanol in Form farbloser Kristalle vom Schmelzpunkt 133-134° ; $[\alpha]_D^{20}$ + 12,9° (c = 1, Aethanol).

d) 2,6 g (7,35 mMol) (+)-(1R)-cis-2-/(S)-4-(Benzyloxy)-α-[(dimethylamino) methyl] benzyl/cyclohexanol werden in 100 ml Aethanol gelöst und nach Versetzen mit 250 mg 5-proz. Palladium/Kohle bei Raumtemperatur hydriert. Nach Abtrennen vom Katalysator wird das Filtrat eingeengt und die erhaltenen Kristalle aus Aethanol umkristallisiert. Man erhält (+)-(1R)-cis-2-/(S)-α-[(Dimethylamino) methyl]-4-hydroxy-benzyl/cyclohexanol in Form farbloser Kristalle vom Schmelzpunkt 235-236° ; $[\alpha]_D^{20} = + 19,8°$ (c = 1, Aethanol).

Beispiel 23

a) Zu einer Lösung von 46,3 g (255,2 mMol) 1-Morpholino-1-cyclohepten in 350 ml Methylenchlorid wird bei 0° innert 45 Minuten unter Rühren eine Lösung von 50,0 g (195,9 mMol) 4-(Benzyloxy)-ω-nitrostyrol in 600 ml Methylenchlorid zugegeben und das Gemisch 3 Stunden bei 0° und anschliessend über Nacht bei Raumtemperatur gerührt. Das Gemisch wird nochmals mit 11,6 g (63,8 mMol) 1-Morpholino-1-cyclohepten versetzt, 5 Stunden bei 0° gerührt und dann unter intensivem Rühren mit 300 ml 1N Salzsäure rasch versetzt und eine Stunde gerührt. Die wässrige Phase wird abgetrennt und zweimal mit je 300 ml Methylenchlorid extrahiert. Die organische Phase wird mit Wasser gewaschen, über Natriumsulfat getrocknet und eingedampft. Das erhaltene Oel wird an 500 g Kieselgel unter Eluieren mit Toluol gereinigt. Nach Umkristallisation aus Isopropyläther erhält man rac-(S*)-2-[(R*)-4-(Benzyloxy)-α-(nitromethyl) benzyl] cycloheptanon in Form farbloser Kristalle vom Schmelzpunkt 120-121°.

b) Zu einer Suspension von 12,2 g (323 mMol) Lithium-aluminiumhydrid in 350 ml trockenem Tetrahydrofuran unter Argon wird unter Rühren eine Lösung von 47,5 g (129,3 mMol) rac-(S*)-2-[(R*)-4-Benzyloxy)-α-(nitromethyl) benzyl] cycloheptanon in 650 ml trockenem Tetrahydrofuran so zugetropft, dass die Temperatur 40° nicht übersteigt. Man rührt über Nacht bei 40°, kühlt ab, versetzt tropfenweise mit 50 ml Tetrahydrofuran/Wasser (1 : 1) und 50 ml konz. Natronlauge, saugt den ausgefallenen Niederschlag unter Waschen mit Methylenchlorid ab und dampft das Filtrat ein. Der ölige Rückstand kristallisiert beim Behandeln mit Isopropyläther. Man erhält rac-(2S*)-[α-(Aminomethyl)-4-(benzyloxy) benzyl] cycloheptanol in Form farbloser Kristalle vom Schmelzpunkt 150-151°.

c) Eine Lösung von 20,0 g (50,91 mMol) rac-[2S*)-α-(Aminomethyl)-4-(benzyloxy) benzyl] cycloheptanol in 180 ml Dimethylformamid wird mit 18 ml 35-proz. Formaldehydlösung und 9,0 ml 88-proz. Ameisensäure während 5 Stunden bei 100° gerührt. Nach dem Abdestillieren der leichtflüchtigen Anteile im Wasserstrahlvakuum bei 70° wird der Rückstand in 200 ml Aether aufgenommen und zweimal mit je 50 ml 3N Salzsäure extrahiert. Die saure Phase wird mit konz. Natronlauge alkalisch gestellt und zweimal mit je 350 ml Methylenchlorid extrahiert. Die organische Phase wird zweimal mit je 100 ml Wasser gewaschen, über Magnesiumsulfat getrocknet und eingedampft. Das erhaltene Oel kristallisiert nach Lösen in Isopropyläther. Erhalten werden 13,7 g rac-(2S*)-/(R*)-4-(Benzyloxy)-α-[(dimethylamino) methyl] benzyl/cycloheptanol in Form farbloser Kristalle vom Schmelzpunkt 106-107°.

d) 12,0 g (2S*)-/(R*)-4-(Benzyloxy)-α-[(dimethylamino)-methyl] benzyl/cycloheptanol werden in 120 ml Aethanol gelöst und nach Zugabe von 1,2 g 5-proz. Palladium/Kohle bei Raumtemperatur hydriert. Nach Abtrennen vom Katalysator wird das Filtrat eingedampft und der kristalline Rückstand aus Methylenchlorid/Isopropyläther umgelöst. Man erhält rac-(2S*)-/(R*)-α-[(Dimethylamino) methyl]-4-hydroxy-benzyl/cycloheptanol in Form farbloser Kristalle vom Schmelzpunkt 197-198°. Das auf übliche Art bereitete rac-(2S*)-/(R*)-α-[(Dimethylamino) methyl]-4-hydroxybenzyl/cycloheptanol-hydrochlorid kristallisiert nach Versetzen mit Aether und schmilzt bei 66-68°.

Beispiel 24

a) Eine Lösung von 27,5 g (272,5 mMol) Diisopropylamin in 250 ml trockenem Tetrahydrofuran wird auf —78° abgekühlt und bei dieser Temperatur innert 30 Minuten unter Rühren tropfenweise mit 170 ml einer 1,6 molaren Butyllithium/Hexanlösung (272,5 mMol) versetzt. Nach 15 Minuten wird innert 10 Minuten eine Lösung von 34,5 g (272,5 mMol) Cyclooctanon in 250 ml trockenem Tetrahydrofuran zugegeben und das Gemisch während 1 Stunde bei —78° weitergerührt. Anschliessend wird eine Lösung von 50 g (272,5 mMol) 4-Chlor-ω-nitrostyrol in 500 ml trockenem Tetrahydrofuran innert 45 Minuten zugegeben und während 2 Stunden bei —78° weitergerührt. Dann versetzt man das Reaktionsgemisch mit 27,5 ml Eisessig, giesst das Ganze auf 1 l Eiswasser und extrahiert 3-mal mit je 500 ml Methylenchlorid. Die organische Phase wird mit Wasser gewaschen, über Natriumsulfat getrocknet und das Lösungsmittel abdestilliert. Das erhaltene Oel (90 g) wird an der 10-fachen Menge Kieselgel mit Toluol chromatographiert. Man erhält rac-(2S*)-2-[4-Chlor-α-(nitromethyl) benzyl] cyclooctanon in Form eines gelblichen Oels.

b) Zu einer Suspension von 2,3 g (59,7 mMol) Lithium-aluminiumhydrid in 75 ml trockenem Tetrahydrofuran unter Argon wird eine Lösung von 7,4 g (23,9 mMol) rac-(2S*)-2-[4-Chlor-α-(nitromethyl) benzyl] cyclooctanon in 100 ml trockenem Tetrahydrofuran unter Rühren bei 40° zugetropft und über

Nacht unter Rückflusstemperatur weitergerührt. Man kühlt das Gemisch ab, zersetzt überschüssiges Lithium-aluminiumhydrid mit Tetrahydrofuran/Wasser (1 : 1), saugt den ausgefallenen Niederschlag unter Waschen mit Methylenchlorid ab und dampft das Filtrat ein. Der Rückstand wird in Aether gelöst, die ätherische Phase mit 1N Salzsäure extrahiert, die saure Phase mit konz. Ammoniak alkalisch gestellt und mit Methylenchlorid extrahiert. Die organische Phase wird mit Wasser gewaschen, über Magnesiumsulfat getrocknet und eingedampft. Nach Kristallisation aus Methylenchlorid/Isopropyläther erhält man rac-(2S*)-2-[(R*)-α-(Aminomethyl)-4-chlorbenzyl] cyclooctanol in Form farbloser Kristalle vom Schmelzpunkt 110-111°.

c) Man rührt eine Lösung von 3,0 g rac-(2S*)-2-[(R*)-α-(Aminomethyl)-4-chlorbenzyl] cyclooctanol in einem Gemisch aus 25 ml Dimethylformamid, 2,0 ml 35-proz. Formaldehydlösung und 1,2 ml 88-proz. Ameisensäure während 2 Stunden bei 100°, destilliert die leichtflüchtigen Anteile bei 70° am Wasserstrahlvakuum ab, versetzt den Rückstand mit konz. Ammoniak und extrahiert mit Methylenchlorid. Die organische Phase wird einmal mit 50 ml Wasser gewaschen, über Natriumsulfat getrocknet und das Lösungsmittel abgedampft. Das erhaltene Oel wird an der 10-fachen Menge Aluminiumoxid mit Toluol gereinigt. Nach Elution mit Toluol erhält man rac-(2S*)-2-/(R*)-4-Chlor-α-[(dimethylamino) methyl] benzyl/-cyclooctanol in Form eines farblosen Oels, das auf übliche Art in das Hydrochlorid übergeführt wird. Nach Kristallisation aus Alkohol/Aether erhält man rac-(2S*)-2-/(R*)-4-Chlor-α-[(dimethylamino) methyl] benzyl/cyclooctanol-hydrochlorid in Form farbloser Kristalle vom Schmelzpunkt 252-253°.

## Beispiel 25

a) Zu einer Lösung von 21,8 g (142 mMol) N-(1-Cyclopenten-1-yl) morpholin in 200 ml Methylenchlorid wird unter Rühren und Kühlen auf 0° eine Lösung von 20,0 g (109 mMol) 4-Chlor-ω-nitrostyrol in 300 ml Methylenchlorid innert etwa 30 Minuten zugetropft und das Gemisch während 3 Stunden bei 0° weitergerührt. Das Gemisch wird mit 142 ml 1N Salzsäure versetzt und noch 1 Stunde gerührt. Die wässrige Phase wird abgetrennt und zweimal mit je 100 ml Methylenchlorid extrahiert. Die oganische Phase wird mit Wasser gewaschen, über Natriumsulfat getrocknet und das Lösungsmittel abdestilliert. Nach Chromatographie an Kieselgel eluiert man mit Toluol 2-[4-Chlor-α-(nitromethyl) benzyl] cyclopentanon als gelbliches Oel (Diastereomerengemisch im Verhältnis 70 : 30).

b) Zu einer Suspension von 5,0 g (132,6 mMol) Lithium-aluminiumhydrid in 150 ml trockenem Tetrahydrofuran wird unter Argon eine Lösung von 14,2 g (53 mMol) 2-[4-Chlor-α-(nitromethyl) benzyl] cyclopentanon in 200 ml trockenem Tetrahydrofuran innert 1 Stunde zugetropft und das Gemisch über Nacht zum Rückfluss erwärmt. Nach dem Abkühlen wird überschüssiges Lithiumaluminiumhydrid mit Tetrahydrofuran/-Wasser (1 : 1) und 25 ml konz. Natronlauge zersetzt, der ausgefallene Niederschlag abgesaugt und das Lösungsmittel abdestilliert. Das erhaltene Oel (11,25 g) wird in 100 ml Dimethylformamid gelöst, mit 9,5 ml 37-proz. Formaldehydlösung und 5 ml 88-proz. Ameisensäure versetzt und während 2 Stunden bei 100° gehalten. Nach dem Abkühlen wird der leichtflüchtige Anteil bei 75° im Wasserstrahlvakuum abdestilliert, der Rückstand mit 3N Natronlauge versetzt und mit Methylenchlorid extrahiert. Die organische Phase wird mit Wasser gewaschen, über Natriumsulfat getrocknet und eingedampft. Das erhaltene Oel wird an der 30-fachen Menge Aluminiumoxid mit Toluol chromatographiert. Man erhält öliges 2-/4-Chlor-α-[(dimethylamino) methyl] benzyl/cyclopentanol als Diastereomerengemisch im Verhältnis 3 : 1.

Das auf übliche Art bereitete 2-/4-Chlor-α-[(dimethylamino)-methyl] benzyl/cyclopentanol-hydrochlorid wird aus Alkohol/Aether kristallisiert und schmilzt bei 228-230°.

## Beispiel A

Es werden in üblicher Weise Tabletten mit Bruchrille folgender Zusammensetzung hergestellt :

| Bestandteile : | mg/Tablette |
|---|---|
| rac-(1S*)-cis-2-/(R*)-4-Chlor-α-[(dimethylamino) methyl] benzyl/-cyclohexanol | 50,0 |
| Milchzucker | 102,0 |
| Maisstärke | 45,0 |
| Talk | 10,4 |
| Magnesiumstearat | 2,6 |
| Tablettengewicht | 210,0 |

**Patentansprüche** (für die Vertragsstaaten : BE, CH, DE, FR, GB, IT, LI, LU, NL, SE)

1. Verbindungen der allgemeinen Formel

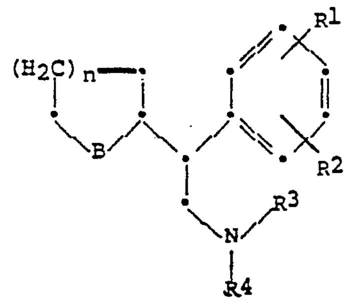

(I)

worin $R^1$ und $R^2$ je Wasserstoff, Halogen, Trifluormethyl, niederes Alkoxy, niederes Alkyl, Hydroxy oder Nitro bedeuten, wobei mindestens einer von $R^1$ und $R^2$ von Wasserstoff verschieden ist, $R^3$ und $R^4$ je niederes Alkyl, n die Zahl 1, 2, 3 oder 4 und B die Gruppe —CO— oder —CHOH— bedeuten, und pharmazeutisch annehmbare Säureadditionssalze davon.

2. Verbindungen gemäss Anspruch 1, worin n die Zahl 1, 2 oder 3 bedeutet.

3. Verbindungen gemäss Anspruch 1 oder 2 mit der in der Formel Ia oder Ib gezeigten relativen Konfiguration und die entsprechenden optisch einheitlichen enantiomeren Formen :

(IV)

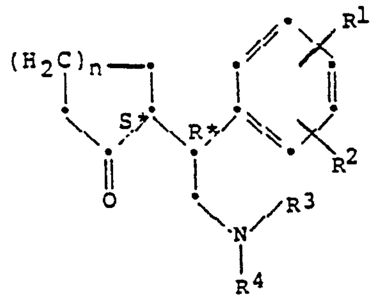

(Ia)

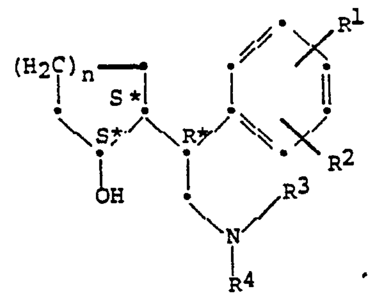

(Ib)

4. Verbindungen gemäss einem der Ansprüche 1-3, worin entweder $R^1$ Halogen, niederes Alkoxy oder Hydroxy und $R^2$ Wasserstoff oder $R^1$ und $R^2$ jeweils beide Halogen, niederes Alkoxy oder Hydroxy bedeuten.

5. Verbindungen gemäss einem der Ansprüche 1-4, worin n die Zahl 2 bedeutet.

6. Verbindungen gemäss einem der Ansprüche 1-5, worin B die Gruppe —CHOH— bedeutet.

7. Verbindungen gemäss einem der Ansprüche 1-6, worin $R^3$ und $R^4$ Methyl bedeuten.

8. rac-(1S*)-cis-2-/(R*)-4-Chlor-α-[(dimethylamino)-methyl] benzyl/cyclohexanol.

9. rac-(1S*)-cis-2-/(R*)-α-[(Dimethylamino) methyl]-4-hydroxybenzyl/cyclohexanol.

10. (1S)-cis-2-/(R)-4-Chlor-α-[(dimethylamino) methyl]-benzyl/cyclohexanol.

11. (1R)-cis-2-/(S)-4-Chlor-α-[(dimethylamino) methyl]-benzyl/cyclohexanol.

12. rac-(1S*)-cis-2-/(R*)-3,4-Dichlor-α-[(dimethylamino) methyl] benzyl/cyclohexanol.

13. rac-(1S*)-cis-2-/(R*)-4-Methoxy-α-[(dimethylamino) methyl] benzyl/cyclohexanol.

14. rac-(1S*)-cis-2-/(R*)-α-[(Dimethylamino) methyl]-3,4-dimethoxybenzyl] cyclohexanol.

15. rac-(2S*)-2-/(R*)-4-Chlor-α-[(dimethylamino) methyl]-benzyl/cyclohexanon.

16. (1S)-cis-2/(R)-α-[(Dimethylamino) methyl]-4-hydroxybenzyl/cyclohexanol.

17. Verbindungen der allgemeinen Formel

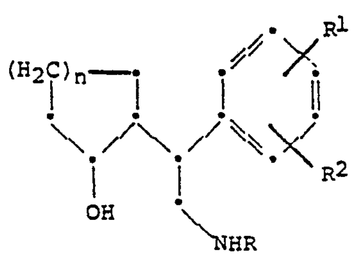

(II)

worin $R^1$ und $R^2$ je Wasserstoff, Halogen, Trifluormethyl, niederes Alkoxy, niederes Alkyl, Hydroxy oder Nitro bedeuten, wobei mindestens einer von Wasserstoff verschieden ist, R Wassertoff oder niederes Alkyl und n die Zahl 1, 2, 3 oder 4 bedeuten.

18. Verbindungen der allgemeinen Formel

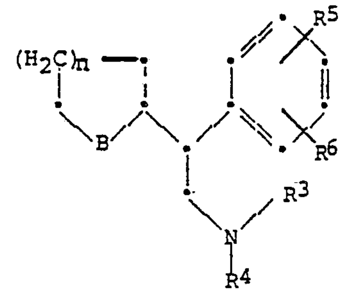

(III)

worin $R^3$ und $R^4$ je niederes Alkyl, n die Zahl 1, 2, 3 oder 4 und B die Gruppe —CO— oder —CHOH— bedeuten, und einer der Reste $R^5$ und $R^6$ eine geschützte Hydroxylgruppe und der andere Wasserstoff, Halogen, Trifluormethyl, niederes Alkoxy, niederes Alkyl, Nitro oder eine geschützte Hydroxylgruppe darstellt, mit der Massgabe, dass der Ausdruck geschützte Hydroxylgruppe nicht niederes Alkoxy bezeichnet.

19. Verbindungen der allgemeinen Formel

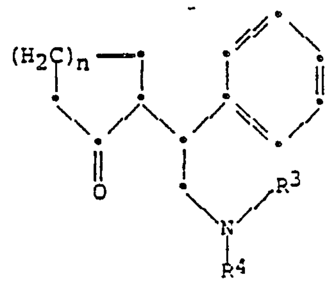

(IV)

worin $R^3$ und $R^4$ je niederes Alkyl und n die Zahl 1, 2, 3 oder 4 bedeuten.

20. Verbidungen gemäss einem der Ansprüche 1-16 zur Anwendung als pharmazeutische Wirkstoffe.

21. Verbindungen gemäss einem der Ansprüche 1-16 zur Anwendung als analgetisch und antidepressiv wirksame Stoffe.

22. Verfahren zur Herstellung von Verbindungen gemäss einem der Ansprüche 1-16, dadurch gekennzeichnet, dass man

a) in eine Verbindung der allgemeinen Formel

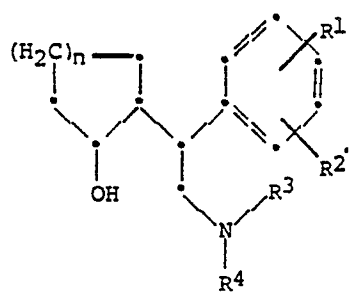

(II)

worin $R^1$, $R^2$, und n die in Anspruch 1 angegebene Bedeutung besitzen, und R Wasserstoff oder niederes Alkyl bedeutet, die primäre oder sekundäre Aminogruppe di- bzw. mono-alkyliert, oder

b) in einer Verbindung der allgemeinen Formel

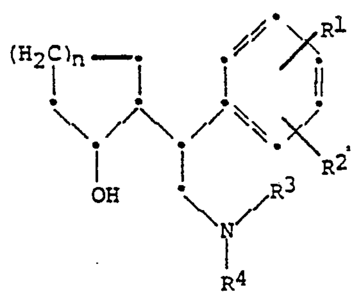

(Ic)

worin $R^1$, $R^2$, $R^3$, $R^4$ und n die in Anspruch 1 angegebene Bedeutung besitzen, die sekundäre Alkoholgruppierung oxidiert, oder

c) aus einer Verbindung der allgemeinen Formel

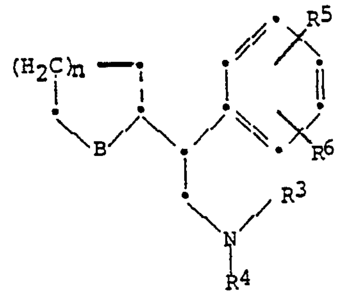

(III)

worin $R^3$, $R^4$, n und B die in Anspruch 1 angegebene Bedeutung besitzen, und einer der Reste $R^5$ und $R^6$ eine geschützte Hydroxylgruppe und der andere Wasserstoff, Halogen, Trifluormethy, niederes Alkoxy, niederes Alkyl, Nitro oder eine geschützte Hydroxylgruppe bedeutet, die Schutzgruppe(n) abspaltet, oder

d) eine Verbindung der allgemeinen Formel

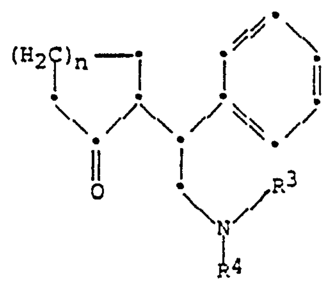

worin $R^3$, $R^4$ und n die in Anspruch 1 angegebene Bedeutung besitzen, in der p-Stellung des Phenylringes nitriert, und erwünschtenfalls

e) ein erhaltenes Gemisch von unterschiedlichen Racematen in die Racemate auftrennt,

f) ein erhaltenes Racemat in die optischen Antipoden spaltet und/oder

g) eine erhaltene Verbindung der Formel I in ein pharmazeutisch annehmbares Säureadditionssalz überführt.

23. Arzneimittel, enthaltend eine Verbindung gemäss einem der Ansprüche 1-16 und ein therapeutisch inertes Excipiens.

24. Verwendung einer Verbindung gemäß einem der Ansprüche 1-16 zur Herstellung eines analgetisch und antidepressiv wirksamen Arzneimittels.

**Patentansprüche** (für den Vertragsstaat AT)

1. Verbindungen der allgemeinen Formel

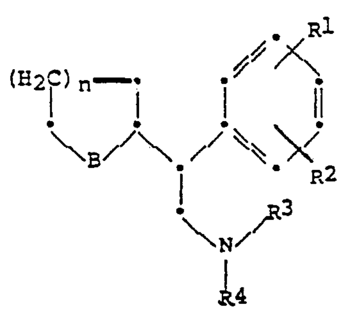

(I)

worin $R^1$ und $R^2$ je Wasserstoff, Halogen, Trifluormethyl, niederes Alkoxy, niederes Alkyl, Hydroxy oder Nitro bedeuten, wobei mindestens einer von $R^1$ und $R^2$ von Wasserstoff verschieden ist, $R^3$ und $R^4$ je niederes Alkyl, n die Zahl 1, 2, 3 oder 4 und B die Gruppe —CO— oder —CHOH— bedeuten, und pharmazeutisch annehmbare Säureadditionssalze davon.

2. Verbindungen gemäss Anspruch 1, worin n die Zahl 1, 2 oder 3 bedeutet.

3. Verbindungen gemäss Anspruch 1 oder 2 mit der in der Formel Ia oder Ib gezeigten relativen Konfiguration und die entsprechenden optisch einheitlichen enantiomeren Formen :

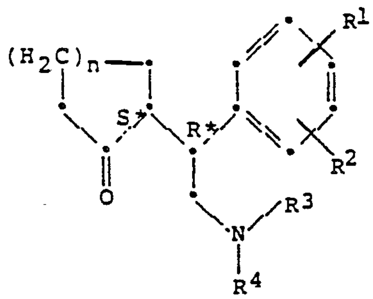
(Ia)

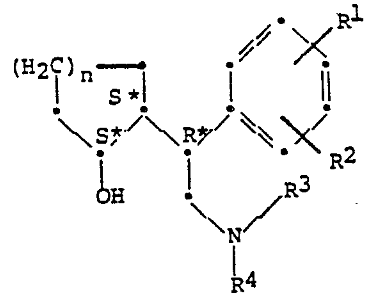
(Ib)

4. Verbindungen gemäss einem der Ansprüche 1-3, worin entweder $R^1$ Halogen, niederes Alkoxy oder Hydroxy und $R^2$ Wasserstoff oder $R^1$ und $R^2$ jeweils beide Halogen, niederes Alkoxy oder Hydroxy bedeuten.

5. Verbindungen gemäss einem der Ansprüche 1-4, worin n die Zahl 2 bedeutet.

6. Verbindungen gemäss einem der Ansprüche 1-5, worin B die Gruppe —CHOH— bedeutet.

7. Verbindungen gemäss einem der Ansprüche 1-6, worin $R^3$ und $R^4$ methyl bedeuten.

8. rac-(1S*)-cis-2-/(R*)-4-Chlor-α-[(dimethylamino)-methyl]benzyl/cyclohexanol.

9. rac-(1S*)-cis-2-/(R*)-α-[(Dimethylamino) methyl]-4-hydroxybenzyl/cyclohexanol.

10. (1S)-cis-2-/(R)-4-Chlor-α[(dimethylamino) methyl]-benzyl/cyclohexanol.

11. (1R)-cis-2-/(S)-4-Chlor-α-[(dimethylamino) methyl]-benzyl/cyclohexanol.

12. rac-(1S*)-cis-2-/(R*)-3,4-Dichlor-α-[(dimethylamino) methyl] benzyl/cyclohexanol.

13. rac-(1S*)-cis-2-/R*)-4-Methoxy-α-[(dimethylamino) methyl] benzyl/cyclohexanol.

14. rac-(1S*)-cis-2-/(R*)-α-[(Dimethylamino) methyl]-3,4-dimethoxybenzyl] cyclohexanol.

15. rac-(2S*)-2-/(R*)-4-Chlor-α-[(dimethylamino) methyl]-benzyl/cyclohexanon.

16. (1S)-cis-2-/(R)-α-[(Dimethylamino)methyl]-4-hydroxybenzyl/cyclohexanol.

17. Verbindungen der allgemeinen Formel

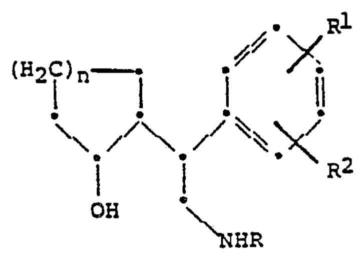
(II)

worin $R^1$ und $R^2$ je Wasserstoff, Halogen, Trifluormethyl, niederes Alkoxy, niederes Alkyl, Hydroxy oder Nitro bedeuten, wobei mindestens einer von Wasserstoff verschieden ist, R Wasserstoff oder niederes Alkyl und n die Zahl 1, 2, 3 oder 4 bedeuten.

18. Verbindungen der allgemeinen Formel

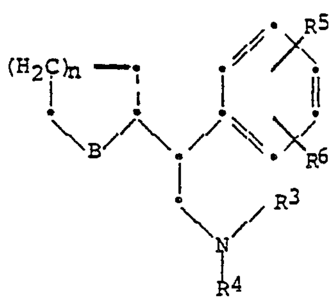
(III)

worin $R^3$ und $R^4$ je niederes Alkyl, n die Zahl 1, 2, 3 oder 4 und B die Gruppe —CO— oder —CHOH— bedeuten, und einer der Reste $R^5$ und $R^6$ eine geschützte Hydroxylgruppe und der andere Wasserstoff, Halogen, Trifluormethyl, niederes Alkoxy, niederes Alkyl, Nitro oder eine geschützte Hydroxylgruppe darstellt, mit der Massgabe, dass der Ausdruck geschützte Hydroxylgruppe nicht niederes Alkoxy bezeichnet.

19. Verbindungen der allgemeinen Formel

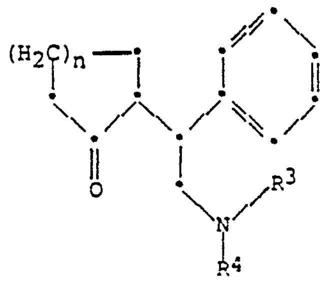

(IV)

worin $R^3$ und $R^4$ je niederes Alkyl und n die Zahl 1, 2, 3 oder 4 bedeuten.

20. Verbindungen gemäss einem der Ansprüche 1-16 zur Anwendung als pharmazeutische Wirkstoffe.

21. Verbindungen gemäss einem der Ansprüche 1-16 zur Anwendung als analgetisch und antidepressiv wirksame Stoffe.

22. Verfahren zur Herstellung von Verbindungen der allgemeinen Formel

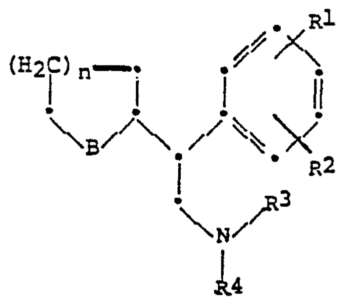

(I)

worin $R^1$ und $R^2$ je Wasserstoff, Halogen, Trifluormethyl, niederes Alkoxy, niederes Alkyl, Hydroxy oder Nitro bedeuten, wobei mindestens einer von $R^1$ und $R^2$ von Wasserstoff verschieden ist, $R^3$ und $R^4$ je niederes Alkyl, n die Zahl 1, 2, 3 oder 4 und B die Gruppe —CO— oder —CHOH— bedeuten, und pharmazeutisch annehmbaren Säureadditionssalzen davon, dadurch gekennzeichnet, dass man

a) in eine Verbindung der allgemeinen Formel

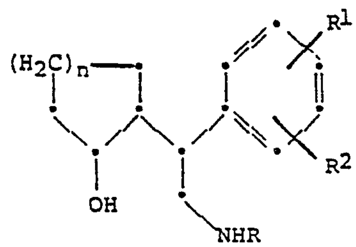

(II)

worin $R^1$, $R^2$, und n die obige Bedeutung besitzen, und R Wasserstoff oder niederes Alkyl bedeutet, die primäre oder sekundäre Aminogruppe di- bzw. monoalkyliert, oder

b) in einer Verbindung der allgemeinen Formel

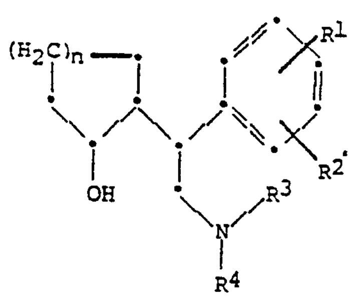

(Ic)

worin $R^1$, $R^2$, $R^3$, $R^4$ und n die obige Bedeutung besitzen, die sekundäre Alkoholgruppierung oxidiert, oder

c) aus einer Verbindung der allgemeinen Formel

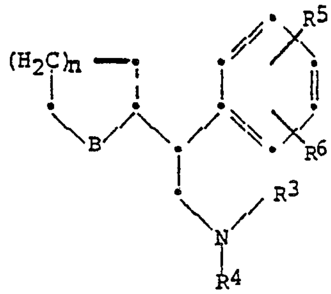

(III)

worin $R^3$, $R^4$, n und B die obige Bedeutung besitzen, und einer der Reste $R^5$ und $R^6$ eine geschützte Hydroxylgruppe und der andere Wasserstoff, Halogen, Trifluormethyl, niederes Alkoxy, niederes Alkyl, Nitro oder eine geschützte Hydroxylgruppe bedeutet, die Schutzgruppe(n) abspaltet, oder

d) eine Verbindung der allgemeinen Formel

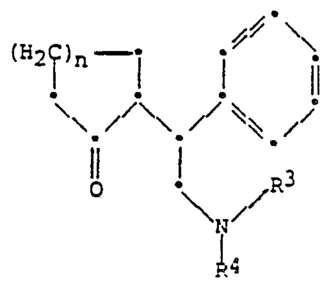

(IV)

worin $R^3$, $R^4$ und n die obige Bedeutung besitzen, in der p-Stellung des Phenylringes nitriert, und erwünschtenfalls

e) ein erhaltenes Gemisch von unterschiedlichen Racematen in die Racemate auftrennt,

f) ein erhaltenes Racemat in die optischen Antipoden spaltet und/oder

g) eine erhaltene Verbindung der Formel I in ein pharmazeutisch annehmbares Säureadditionssalz überführt.

23. Verfahren gemäss Anspruch 22, worin n die Zahl 1, 2 oder 3 bedeutet.

24. Verfahren gemäss Anspruch 22 oder 23, dadurch gekennzeichnet, dass man eine Verbindung mit der in der Formel Ia oder Ib gezeigten relativen Konfiguration oder die entsprechenden optisch einheitlichen enantiomeren Formen herstellt :

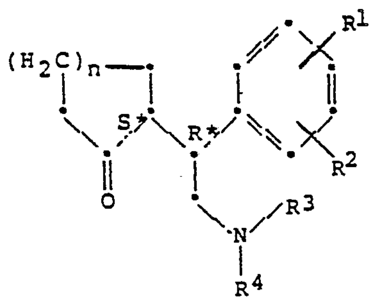

(Ia)

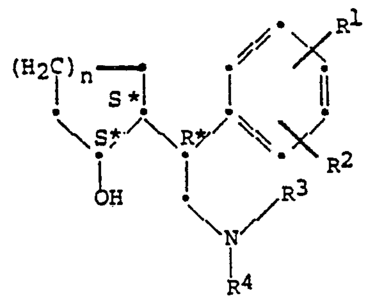

(Ib)

25. Verfahren gemäss einem der Ansprüche 22-24, dadurch gekennzeichnet, dass man eine Verbindung der Formel I herstellt, worin entweder $R^1$ Halogen, niederes Alkoxy oder Hydroxy und $R^2$ Wasserstoff oder $R^1$ und $R^2$ jeweils beide Halogen, niederes Alkoxy oder Hydroxy bedeuten.

26. Verfahren gemäss einem der Ansprüche 22-25, dadurch gekennzeichnet, dass man eine Verbindung der Formel I herstellt, worin n die Zahl 2 bedeutet.

27. Verfahren gemäss einem der Ansprüche 22-26, dadurch gekennzeichnet, dass man eine Verbindung der Formel I herstellt, worin B die Gruppe —CHOH— bedeutet.

28. Verfahren gemäss einem der Ansprüche 22-27, dadurch gekennzeichnet, dass man eine Verbindung der Formel I herstellt, worin $R^3$ und $R^4$ Methyl bedeuten.

29. Verfahren gemäss Anspruch 22 oder 23, dadurch gekennzeichnet, dass man rac-(1S*)-cis-2-/(R*)-4-Chlor-α-[(dimethylamino) methyl] benzyl/cyclohexanol herstellt.

30. Verfahren gemäss Anspruch 22 oder 23, dadurch gekennzeichnet, dass man rac-(1S*)-cis-2-/(R*)-α-[(Dimethylamino) methyl]-4-hydroxybenzyl/cyclohexanol herstellt.

31. Verfahren gemäss Anspruch 22 oder 23, dadurch gekennzeichnet, dass man (1S)-cis-2-/(R)-4-Chlor-α-[(dimethylamino) methyl] benzyl/cyclohexanol herstellt.

32. Verfahren gemäss Anspruch 22 oder 23, dadurch gekennzeichnet, dass man (1R)-cis-2-/(S)-4-Chlor-α-[(dimethylamino) methyl] benzyl/cyclohexanol herstellt.

33. Verfahren gemäss Anspruch 22 oder 23, dadurch gekennzeichnet, dass man rac-(1S*)-cis-2-/(R*)-3,4-Dichlor-α-[(dimethylamino) methyl] benzyl/cyclohexanol herstellt.

34. Verfahren gemäss Anspruch 22 oder 23, dadurch gekennzeichnet, dass man rac-(1S*)-cis-2-/(R*)-4-Methoxy-α-[(dimethylamino) methyl] benzyl/cyclohexanol herstellt.

35. Verfahren gemäss Anspruch 22 oder 23, dadurch gekennzeichnet, dass man rac-(1S*)-cis-2-/(R*)-α-[(Dimethylamino) methyl]-3,4-dimethoxybenzyl] cyclohexanol herstellt.

36. Verfahren gemäss Anspruch 22 oder 23, dadurch gekennzeichnet, dass man rac-(2S*)-2-/(R*)-4-Chlor-α-[(dimethylamino) methyl] benzyl/cyclohexanon herstellt.

37. Verfahren gemäss Anspruch 22 oder 23, dadurch gekennzeichnet, dass man (1S)-cis-2-/(R)-α-[(Dimethylamino)-methyl]-4-hydroxybenzyl/cyclohexanol herstellt.

38. Arzneimittel, enthaltend eine Verbindung gemäss einem der Ansprüche 1-16 und ein therapeutisch inertes Excipiens.

39. Verwendung einer Verbindung gemäß einem der Ansprüche 1-16 zur Herstellung eines analgetisch und anti-depressiv wirksamen Arzneimittels.

**Claims** (for the Contracting States : BE, CH, DE, FR, GB, IT, LI, LU, NL, SE)

1. Compounds of the general formula

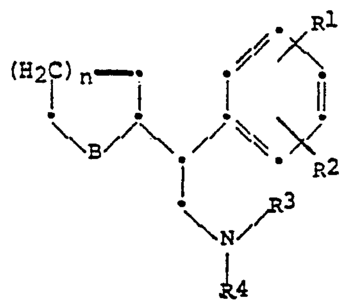

(I)

wherein $R^1$ and $R^2$ each signify hydrogen, halogen, trifluoromethyl, lower alkoxy, lower alkyl, hydroxy or nitro, whereby at least one of $R^1$ and $R^2$ is different from hydrogen, $R^3$ and $R^4$ each signify lower alkyl, n signifies the number 1, 2, 3 or 4 and B signifies the group —CO— or —CHOH—, and pharmaceutically acceptable acid addition salts thereof.

2. Compounds in accordance with claim 1, wherein n signifies the number 1, 2 or 3.

3. Compounds in accordance with claim 1 or 2 with the relative configuration depicted in formula Ia or Ib and the corresponding optically uniform enantiomeric forms :

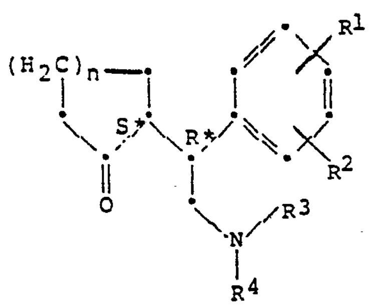

(Ia)

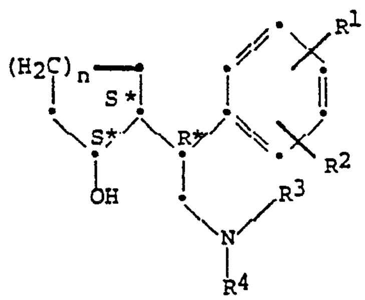

(Ib)

4. Compounds in accordance with any one of claims 1-3, wherein either $R^1$ signifies halogen, lower alkoxy or hydroxy and $R^2$ signifies hydrogen or $R^1$ and $R^2$ in each case both signify halogen, lower alkoxy or hydroxy.

5. Compounds in accordance with any one of claims 1-4, wherein n signifies the number 2.

6. Compounds in accordance with any one of claims 1-5, wherein B signifies the group —CHOH—.

7. Compounds in accordance with any one of claims 1-6, wherein $R^3$ and $R^4$ signify methyl.

8. rac-(1S*)-cis-2-/(R*)-4-Chloro-α-[(dimethylamino) methyl] benzyl/cyclohexanol.

9. rac-(1S*)-cis-2/(R*)-α-[(Dimethylamino) methyl]-4-hydroxybenzyl/cyclohexanol.

10. (1S)-cis-2-/(R)-4-chloro-α-[(dimethylamino)-methyl] benzyll/cyclohexanol.

11. (1R)-cis-2-/(S)-4-Chloro-α-[(dimethylamino)-methyl] benzyl/cyclohexanol.

12. rac-(1S*)-cis-2-/(R*)-3,4-Dichloro-α-[(dimethylamino) methyl] benzyl/cyclohexanol.
13. rac-(1S*)-cis-2-/(R*)-4-Methoxy-α-[(dimethylamino) methyl] benzyl/cyclohexanol.
14. rac-(1S*)-cis-2-/(R*)-α-[(Dimethylamino) methyl]-3,4-dimethoxybenzyl] cyclohexanol.
15. rac-(2S*)-2-/(R*)-4-Chloro-α-[(dimethylamino)-methyl] benzyl/cyclohexanone.
16. (1S)-cis-2-/(R)-α-[(Dimethylamino) methyl]-4-hydroxybenzyl/cyclohexanol.
17. Compounds of the general formula

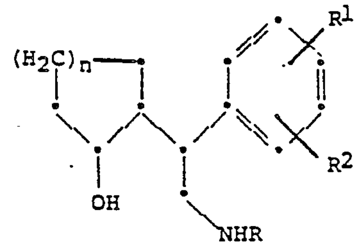

(II)

wherein $R^1$ and $R^2$ each signify hydrogen, halogen, trifluoromethyl, lower alkyl, hydroxy or nitro, whereby at least one is different from hydrogen, R signifies hydrogen or lower alkyl and n signifies the number 1, 2, 3 or 4.

18. Compounds of the general formula

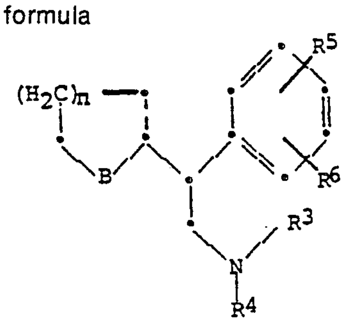

(III)

wherein $R^3$ and $R^4$ each signify lower alkyl, n signifies the number 1, 2, 3 or 4 and B signifies the group —CO— or —CHOH—, and one of the residues $R^5$ and $R^6$ represents a protected hydroxyl group and the other represents hydrogen, halogen, trifluoromethyl, lower alkoxy, lower alkyl, nitro or a protected hydroxyl group, with the proviso that the term protected hydroxyl group does not denote lower alkoxy.

19. Compounds of the general formula

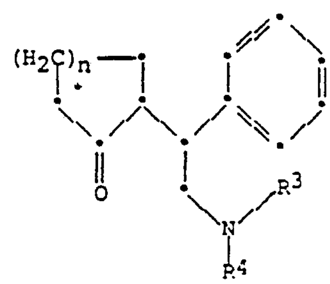

(IV)

wherein $R^3$ and $R^4$ each signify lower alkyl and n signifies the number 1, 2, 3 or 4.

20. Compounds in accordance with any one of claims 1-16 for use as pharmaceutically active substances.

21. Compounds in accordance with any one of claims 1-16 for use as substances having analgesic and antidepressant activity.

22. A process for the manufacture of compounds in accordance with any one of claims 1-16, characterized by

a) di- or mono-alkylating the primary or secondary amino group in a compound of the general formula

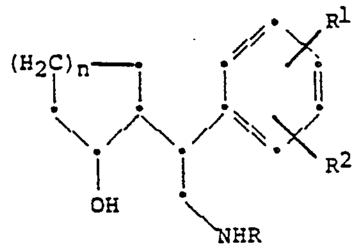

(II)

wherein $R^1$, $R^2$ and n have the significance given in claim 1 and R signifies hydrogen or lower alkyl, or

b) oxidizing the secondary alcohol grouping in a compound of the general formula

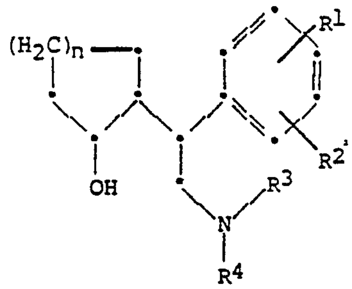

(Ic)

wherein $R^1$, $R^2$, $R^3$, $R^4$ and n have the significance given in claim 1, or

c) cleaving off the protecting group(s) from a compound of the general formula

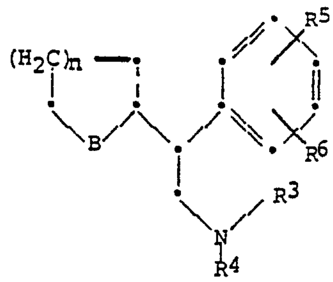

(III)

wherein $R^3$, $R^4$, n and B have the significance given in claim 1 and one of the residues $R^5$ and $R^6$ signifies a protected hydroxyl group and the other signifies hydrogen, halogen, trifluoromethyl, lower alkoxy, lower alkyl, nitro or a protected hydroxyl group, or

d) nitrating a compound of the general formula

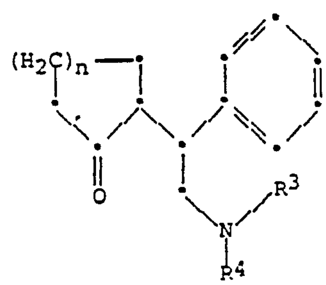

(IV)

wherein $R^3$, $R^4$ and n have the significance given in claim 1, in the p-position of the phenyl ring, and, if desired,

e) separating a mixture of different racemates obtained into the racemates,

f) resolving a racemate obtained into the optical antipodes and/or

g) converting a compound of formula I obtained into a pharmaceutically acceptable acid addition salt.

23. A medicament containing a compound in accordance with any one of claims 1-16 and a therapeutically inert excipient.

24. The use of a compound in accordance with any one of claims 1-16 for the manufacture of a medicament having analgesic and antidepressant activity.

**Claims** (for the Contracting State AT)

1. Compounds of the general formula

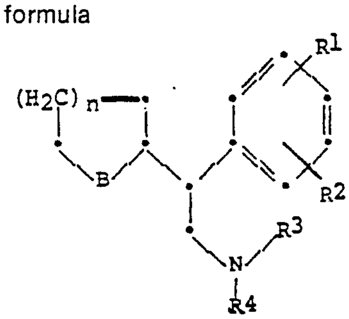

(I)

wherein $R^1$ and $R^2$ each signify hydrogen, halogen, trifluoromethyl, lower alkoxy, lower alkyl, hydroxy or nitro, whereby at least one of $R^1$ and $R^2$ is different from hydrogen, $R^3$ and $R^4$ each signify lower alkyl, n signifies the number 1, 2, 3 or 4 and B signifies the group —CO— or —CHOH—, and pharmaceutically acceptable acid addition salts thereof.

2. Compounds in accordance with claim 1, wherein n signifies the number 1, 2 or 3.

3. Compounds in accordance with claim 1 or 2 with the relative configuration depicted in formula Ia or Ib and the corresponding optically uniform enantiomeric forms :

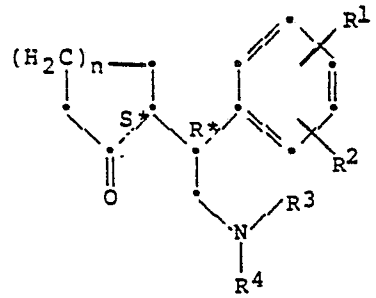
(Ia)

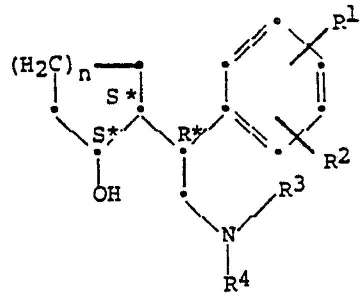
(Ib)

4. Compounds in accordance with any one of claims 1-3, wherein either $R^1$ signifies halogen, lower alkoxy or hydroxy and $R^2$ signifies hydrogen or $R^1$ and $R^2$ in each case both signify halogen, lower alkoxy or hydroxy.

5. Compounds in accordance with any one of claims 1-4, wherein n signifies the number 2.

6. Compounds in accordance with any one of claims 1-5, wherein B signifies the group —CHOH—.

7. Compounds in accordance with any one of claims 1-6, wherein $R^3$ and $R^4$ signify methyl.

8. rac-(1S*)-cis-2-/(R*)-4-Chloro-α-[(dimethylamino) methyl] benzyl/cyclohexanol.

9. rac-(1S*)-cis-2/(R*)-α-[(Dimethylamino) methyl]-4-hydroxybenzyl/cyclohexanol.

10. (1S)-cis-2-/(R)-4-Chloro-α-[(dimethylamino)-methyl] benzyl/cyclohexanol.

11. (1R)-cis-2-/(S)-4-Chloro-α-[(dimethylamino)-methyl] benzyl/cyclohexanol.

12. rac-(1S*)-cis-2-/(R*)-3,4-Dichloro-α-[(dimethylamino) methyl] benzyl/cyclohexanol.

13. rac-(1S*)-cis-2-/(R*)-4-Methoxy-α-[(dimethylamino) methyl] benzyl/cyclohexanol.

14. rac-(1S*)-cis-2-/(R*)-α-[(Dimethylamino) methyl]-3,4-dimethoxybenzyl] cyclohexanol.

15. rac-(2S*)-2-/(R*)-4-Chloro-α-[(dimethylamino)-methyl] benzyl/cyclohexanone.

16. (1S)-cis-2-/(R)-α-[(Dimethylamino) methyl]-4-hydroxybenzyl/cyclohexanol.

17. Compounds of the general formula

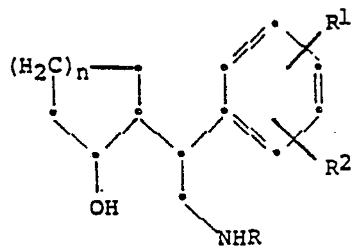
(II)

wherein $R^1$ and $R^2$ each signify hydrogen, halogen, trifluoromethyl, lower alkoxy, lower alkyl, hydroxy or nitro, whereby at least one is different from hydrogen, R signifies hydrogen or lower alkyl and n signifies the number 1, 2, 3 or 4.

18. Compounds of the general formula

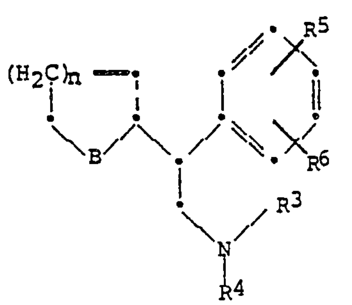
(III)

wherein $R^3$ and $R^4$ each signify lower alkyl, n signifies the number 1, 2, 3 or 4 and B signifies the group —CO— or —CHOH—, and one of the residues $R^5$ and $R^6$ represents a protected hydroxyl group and the other represents hydrogen, halogen, trifluoromethyl, lower alkoxy, lower alkyl, nitro or a protected

hydroxyl group, with the proviso that the term protected hydroxyl group does not denote lower alkoxy.

19. Compounds of the general formula

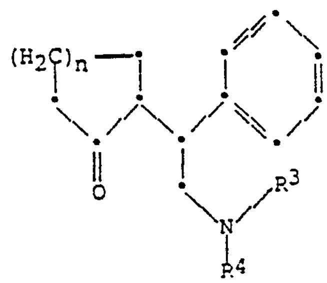

(IV)

wherein $R^3$ and $R^4$ each signify lower alkyl and n signifies the number 1, 2, 3 or 4.

20. Compounds in accordance with any one of claims 1-16 for use as pharmaceutically active substances.

21. Compounds in accordance with any one of claims 1-16 for use as substances having analgesic and antidepressant activity.

22. A process for the manufacture of compounds of the general formula

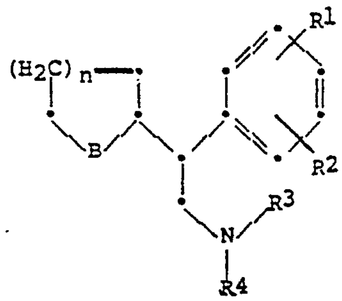

(I)

wherein $R^1$ and $R^2$ each signify hydrogen, halogen, trifluoromethyl, lower alkoxy, lower alkyl, hydroxy or nitro, whereby at least one of $R^1$ and $R^2$ is different from hydrogen, $R^3$ and $R^4$ each signify lower alkyl, n signifies the number 1, 2, 3 or 4 and B signifies the group —CO— or —CHOH—, and pharmaceutically acceptable acid addition salts thereof, characterized by

a) di- or mono-alkylating the primary or secondary amino group in a compound of the general formula

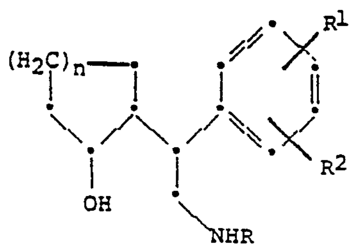

(II)

wherein $R^1$, $R^2$ and n have the above significance and R signifies hydrogen or lower alkyl, or

b) oxidizing the secondary alcohol grouping in a compound of the general formula

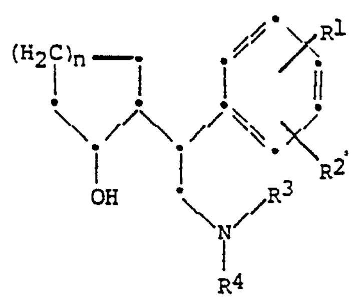

(Ic)

wherein $R^1$, $R^2$, $R^3$, $R^4$ and n have the above significance, or

c) cleaving off the protecting group(s) from a compound of the general formula

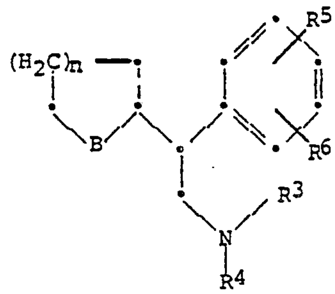

(III)

wherein $R^3$, $R^4$, n and B have the above significance and one of the residues $R^5$ and $R^6$ signifies a protected hydroxyl group and the other signifies hydrogen, halogen, trifluoromethyl, lower alkoxy, lower alkyl, nitro or a protected hydroxyl group, or

d) nitrating a compound of the general formula

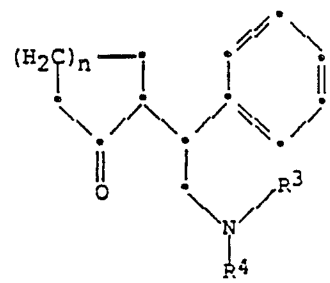

(IV)

wherein $R^3$, $R^4$ and n have the above significance, in the p-position of the phenyl ring, and, if desired,

e) separating a mixture of different racemates obtained into the racemates,

f) resolving a racemate obtained into the optical antipodes and/or

g) converting a compound of formula I obtained into a pharmaceutically acceptable acid addition salt.

23. A process in accordance with claim 22, wherein n signifies the number 1, 2 or 3.

24. A process in accordance with claim 22 or 23, characterized in that there is manufactured a compound with the relative configuration depicted in formula Ia or Ib or the corresponding optically uniform enantiomeric forms :

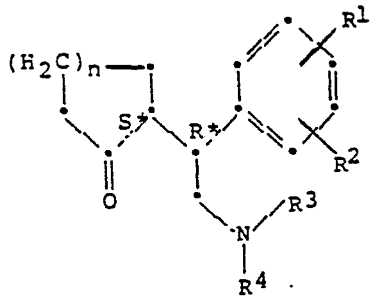

(Ia)

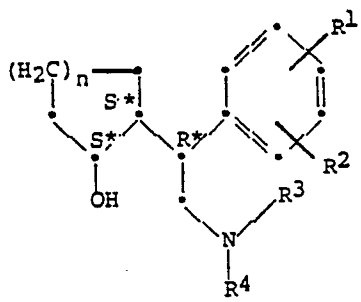

(Ib)

25. A process in accordance with any one of claims 22-24, characterized in that a compound of formula I in which either $R^1$ signifies halogen, lower alkoxy or hydroxy and $R^2$ signifies hydrogen or $R^1$ and $R^2$ in each case both signify halogen, lower alkoxy or hydroxy is manufactured.

26. A process in accordance with any one of claims 22-25, characterized in that a compound of formula I in which n signifies the number 2 is manufactured.

27. A process in accordance with any one of claims 22-26, characterized in that a compound of formula I in which B signifies the group —CHOH— is manufactured.

28. A process in accordance with any one of claims 22-27, characterized in that a compound of formula I in which $R^3$ and $R^4$ signify methyl is manufactured.

29. A process in accordance with claim 22 or 23, characterized in that rac-(1S*)-cis-2-/(R*)-4-chloro-α-[(dimethylamino) methyl] benzyl/cyclohexanol is manufactured.

30. A process in accordance with claim 22 or 23, characterized in that rac-(1S*)-cis-2-/(R*)-α-[(dimethylamino) methyl]-4-hydroxybenzyl/cyclohexanol is manufactured.

31. A process in accordance with claim 22 or 23, characterized in that (1S)-cis-2-/(R)-4-chloro-α-[(dimethylamino) methyl] benzyl/cyclohexanol is manufactured.

32. A process in accordance with claim 22 or 23, characterized in that (1R)-cis-2-/(S)-4-chloro-α-[(dimethylamino) methyl] benzyl/cyclohexanol is manufactured.

33. A process in accordance with claim 22 or 23, characterized in that rac-(1S*)-cis-2-/(R*)-3,4-dichloro-α-[(dimethylamino) methyl] benzyl/cyclohexanol is manufactured.

34. A process in accordance with claim 22 or 23, characterized in that rac-(1S*)-cis-2-/(R*)-4-methoxy-α-[(dimethylamino) methyl] benzyl/cyclohexanol is manufactured.

35. A process in accordance with claim 22 or 23, characterized in that rac-(1S*)-cis-2-/(R*)-α-[(dimethylamino) methyl]-3,4-dimethoxybenzyl] cyclohexanol is manufactured.

36. A process in accordance with claim 22 or 23, characterized in that rac-(2S*)-2-/(R*)-4-chloro-α-[(dimethylamino) methyl] benzyl/cyclohexanone is manufactured.

37. A process in accordance with claim 22 or 23, characterized in that (1S)-cis-2-/(R)-α-[(dimethylamino) methyl]-4-hydroxybenzyl/cyclohexanol is manufactured.

38. A medicament containing a compound in accordance with any one of claims 1-16 and a therapeutically inert excipient.

39. The use of a compound in accordance with any one of claims 1-16 for the manufacture of a medicament having analgesic and antidepressant activity.

**Revendications** (pour les Etats contractants : BE, CH, DE, FR, GB, IT, LI, LU, NL, SE)

1. Composés de formule générale

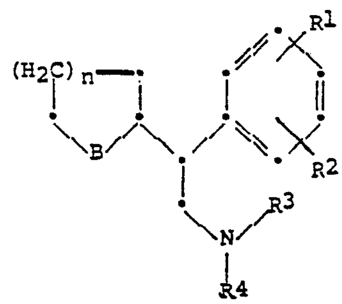

dans laquelle $R^1$ et $R^2$ représentent chacun l'hydrogène, un halogène, le trifluorométhyle, un alcoxy inférieur, alkyle inférieur, hydroxy ou nitro, dans lesquels au moins l'un des deux $R^1$ et $R^2$ n'est pas l'hydrogène, $R^3$ et $R^4$ représentent chacun un alkyle inférieur, n est le nombre 1, 2, 3 ou 4 et B est le groupe —CO— ou —CHOH—, et leurs sels d'addition pharmaceutiquement acceptables.

2. Composés selon la revendication 1, dans lesquels n est le nombre 1, 2 ou 3.

3. Composés selon la revendication 1 ou 2 avec la configuration relative représentée dans la formule Ia ou Ib et les formes énantiomères unitaires optiques correspondantes :

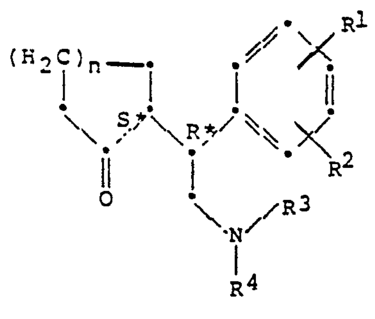
(Ia)

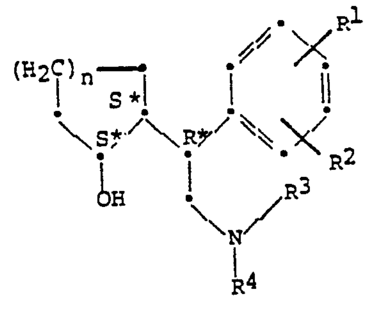
(Ib)

4. Composés selon l'une des revendications 1-3, dans lesquels soit $R^1$ est un halogène, un alcoxy inférieur ou un hydroxy et $R^2$ est l'hydrogène, soit $R^1$ et $R^2$ sont tous les deux un halogène, un alcoxy inférieur ou un hydroxy.

5. Composés selon l'une des revendications 1-4, dans lesquels n est le nombre 2.

6. Composés selon l'une des revendications 1-5, dans lesquels B est le groupe —CHOH—.

7. Composés selon l'une des revendications 1-6, dans lesquels $R^3$ et $R^4$ représentent le méthyle.

8. rac-(1S*)-cis-2-/(R*)-4-chloro-α-[(diméthylamino) méthyl] benzyl/cyclohexanol.

9. rac-(1S*)-cis-2-/(R*)-α-[(diméthylamino)-méthyl]-4-hydroxybenzyl/cyclohexanol.

10. (1S)-cis-2-/(R)-4-chloro-α-[(diméthylamino)-méthyl] benzyl/cyclohexanol.

11. (1R)-cis-2-/(S)-4-chloro-α-[(diméthylamino)-méthyl] benzyl/cyclohexanol.
12. rac-(1S*)-cis-2-/(R*)-3,4-dichloro-α-[(diméthylamino) méthyl] benzyl/cyclohexanol.
13. rac-(1S*)-cis-2-/(R*)-4-méthoxy-α-[(diméthylamino) méthyl] benzyl/cyclohexanol.
14. rac-(1S*)-cis-2-/(R*)-α-[(diméthylamino)-méthyl]-3,4-diméthoxybenzyl] cyclohexanol.
15. rac-(2S*)-2-/(R*)-4-chloro-α-[(diméthylamino) méthyl] benzyl/cyclohexanone.
16. (1S)-cis-2-/(R)-α-[(diméthylamino) méthyl]-4-hydroxybenzyl/cyclohexanol.
17. Composés de formule générale

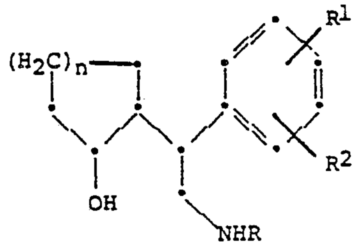

(II)

dans laquelle $R^1$ et $R^2$ représentent chacun l'hydrogène, un halogène, le trifluorométhyle, un alcoxy inférieur, alkyle inférieur, hydroxy ou nitro, dans lesquels au moins l'un des deux n'est pas l'hydrogène, R est l'hydrogène ou un alkyle inférieur et n est le nombre 1, 2, 3 ou 4.

18. Composés de formule générale

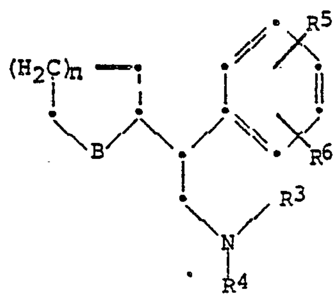

(III)

dans laquelle $R^3$ et $R^4$ représentent chacun un alkyle inférieur, n est le nombre 1, 2, 3 ou 4 et B est le groupe —CO— ou —CHOH—, et l'un des restes $R^5$ et $R^6$ est un groupe hydroxyle protégé et l'autre est un hydrogène, halogène, trifluorométhyle, alcoxy inférieur, alkyle inférieur, nitro ou un groupe hydroxyle protégé avec la condition que l'expression groupe hydroxyle protégé ne désigne pas un alcoxy inférieur.

19. Composés de formule générale

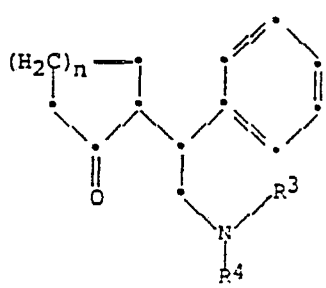

(IV)

dans laquelle $R^3$ et $R^4$ représentent chacun un alkyle inférieur et n est le nombre 1, 2, 3, ou 4.

20. Composés selon l'une des revendications 1-16 pour l'utilisation comme principe actif pharmaceutique.

21. Composés selon l'une des revendications 1-16 pour l'utilisation comme produits à efficacité analgésique et antidépressive.

22. Procédé de préparation des composés selon l'une des revendications 1-16, caractérisé en ce que

a) dans un composé de formule générale

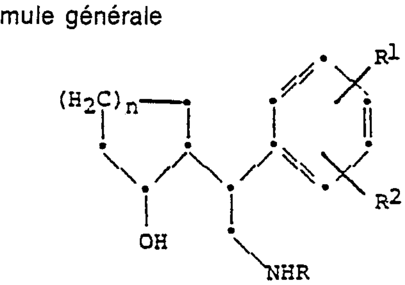

(II)

dans laquelle $R^1$, $R^2$ et n ont la signification donnée dans la revendication 1, et R représente l'hydrogène ou un alkyle inférieur, ou di- ou mono-alkyle respectivement le groupe amino primaire ou secondaire, ou

b) dans un composé de formule générale

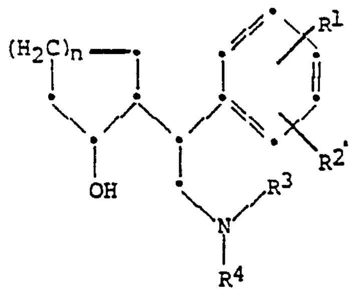

(Ic)

dans laquelle $R^1$, $R^2$, $R^3$, $R^4$ et n ont la signification donnée dans la revendication 1, un oxyde le groupe alcool secondaire, ou

c) dans un composé de formule générale

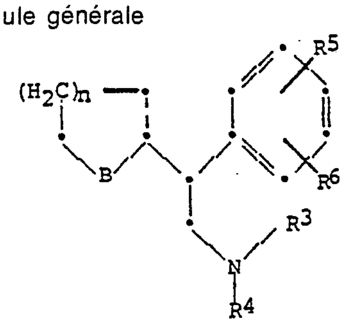

(III)

dans laquelle $R^3$, $R^4$, n et B ont la signification donnée dans la revendication 1, et l'un des restes $R^5$ et $R^6$ est un groupe hydroxyle protégé et l'autre est un hydrogène, halogène, trifluorométhyle, alcoxy inférieur, alkyle inférieur, nitro ou un groupe hydroxyle protégé, on élimine le(s) groupe(s) protecteur(s), ou

d) on nitre en position p du noyau phényle d'un composé de formule générale

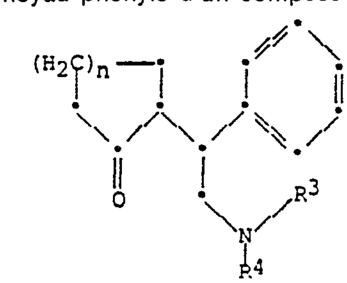

(IV)

dans laquelle $R^3$, $R^4$ et n ont la signification donnée dans la revendication 1 et, si c'est souhaité,

e) on sépare un mélange obtenu de différents racémates en les racémates,

f) on fractionne un racémate obtenu en ses antipodes optiques et/ou

g) on transforme un composé de formule I obtenu en un sel d'addition d'acide pharmaceutiquement acceptable.

23. Médicament contenant un composé selon l'une des revendications 1-16 et un excipient thérapeutiquement inerte.

24. Utilisation d'un composé selon l'une des revendications 1-16 pour préparer un médicament efficace analgésique et antidépresseur.

**Revendications** (pour l'Etat contractant AT)

1. Composés de formule générale

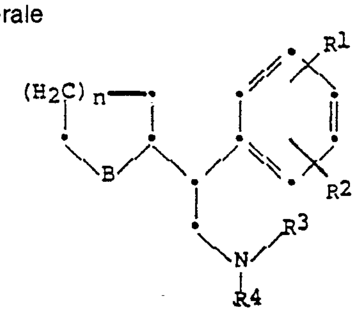

(I)

dans laquelle $R^1$ et $R^2$ représentent chacun l'hydrogène, un halogène, le trifluorométhyle, un alcoxy inférieur, alkyle inférieur, hydroxy ou nitro, dans lesquels au moins l'un des deux $R^1$ et $R^2$ n'est pas l'hydrogène, $R^3$ et $R^4$ représentent chacun un alkyle inférieur, n est le nombre 1, 2, 3 ou 4 et B est le groupe —CO— ou —CHOH—, et leurs sels d'addition pharmaceutiquement acceptables.

2. Composés selon la revendication 1, dans lesquels n est le nombre 1, 2 ou 3.

3. Composés selon la revendication 1 ou 2 avec la configuration relative représentée dans la formule Ia ou Ib et les formes énantiomères unitaires optiques correspondantes :

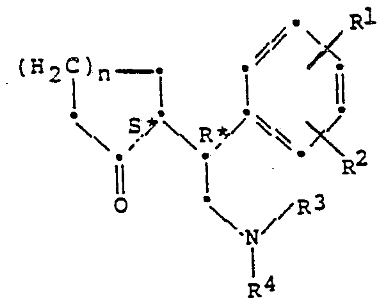
(Ia)

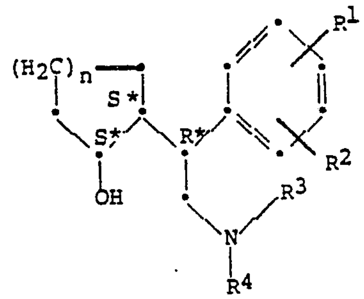
(Ib)

4. Composés selon l'une des revendications 1-3, dans lesquels soit $R^1$ est un halogène, un alcoxy inférieur ou un hydroxy et $R^2$ est l'hydrogène, soit $R^1$ et $R^2$ sont tous les deux un halogène, un alcoxy inférieur ou un hydroxy.

5. Composés selon l'une des revendications 1-4, dans lesquels n est le nombre 2.

6. Composés selon l'une des revendications 1-5, dans lesquels B est le groupe —CHOH—.

7. Composés selon l'une des revendications 1-6, dans lesquels $R^3$ et $R^4$ représentent le méthyle.

8. rac-(1S*)-cis-2-/(R*)-4-chloro-α-[(diméthylamino) méthyl] benzyl/cyclohexanol.

9. rac-(1S*)-cis-2-/(R*)-α-[(diméthylamino)-méthyl]-4-hydroxybenzyl/cyclohexanol.

10. (1S)-cis-2-/(R)-4-chloro-α-[(diméthylamino)-méthyl] benzyl/cyclohexanol.

11. (1R)-cis-2-/(S)-4-chloro-α-[(diméthylamino)-méthyl] benzyl/cyclohexanol.

12. rac-(1S*)-cis-2-/(R*)-3,4-dichloro-α-[(diméthylamino) méthyl] benzyl/cyclohexanol.

13. rac-(1S*)-cis-2-/(R*)-4-méthoxy-α-[(diméthylamino) méthyl] benzyl/cyclohexanol.

14. rac-(1S*)-cis-2-/(R*)-α-[(diméthylamino)-méthyl]-3,4-diméthoxybenzyl] cyclohexanol.

15. rac-(2S*)-2-/(R*)-4-chloro-α-[(diméthylamino) méthyl] benzyl/cyclohexanone.

16. (1S)-cis-2-/(R)-α-[(diméthylamino) méthyl]-4-hydroxybenzyl/cyclohexanol.

17. Composés de formule générale

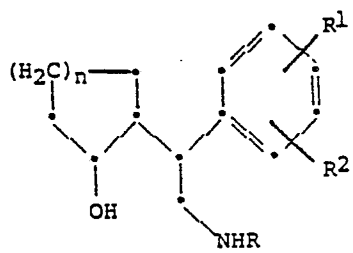
(II)

dans laquelle $R^1$ et $R^2$ représentent chacun l'hydrogène, un halogène, le trifluorométhyle, un alcoxy inférieur, alkyle inférieur, hydroxy ou nitro, dans lesquels au moins l'un des deux n'est pas l'hydrogène, R est l'hydrogène ou un alkyle inférieur et n est le nombre 1, 2, 3 ou 4.

18. Composés de formule générale

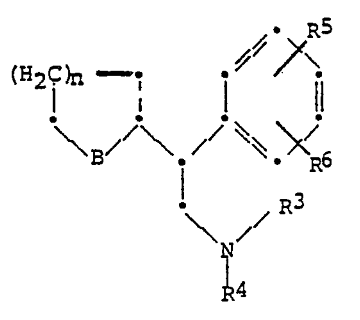
(III)

dans laquelle $R^3$ et $R^4$ représentent chacun un alkyle inférieur, n est le nombre 1, 2, 3 ou 4 et B est le groupe —CO— ou —CHOH—, et l'un des restes $R^5$ et $R^6$ est un groupe hydroxyle protégé et l'autre est un hydrogène, halogène, trifluorométhyle, alcoxy inférieur, alkyle inférieur, nitro ou un groupe hydroxyle

protégé avec la condition que l'expression groupe hydroxyle protégé ne désigne pas un alcoxy inférieur.

19. Composés de formule générale

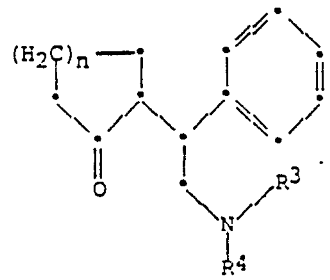

(IV)

dans laquelle $R^3$ et $R^4$ représentent chacun un alkyle inférieur et n est le nombre 1, 2, 3 ou 4.

20. Composés selon l'une des revendications 1-16 pour l'utilisation comme principe actif pharmaceutique.

21. Composés selon l'une des revendications 1-16 pour l'utilisation comme produits à efficacité analgésique et antidépressive.

22. Procédé de préparation de composés de formule générale

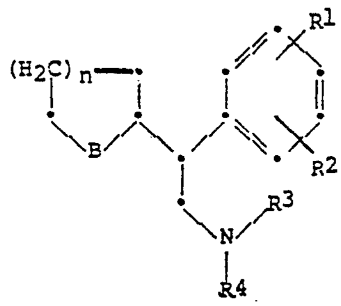

(I)

dans laquelle $R^1$ et $R^2$ représentent chacun l'hydrogène, un halogène, le trifluorométhyle, un alcoxy inférieur, alkyle inférieur, hydroxy ou nitro, dans lesquels au moins l'un des deux $R^1$ ou $R^2$ n'est pas l'hydrogène, $R^3$ et $R^4$ représentent chacun un alkyle inférieur, n est le nombre 1, 2, 3 ou 4 et B est le groupe —CO— ou —CHOH—, et leurs sels d'addition d'acide pharmaceutiquement acceptables, caractérisé en ce que

a) dans un composé de formule générale

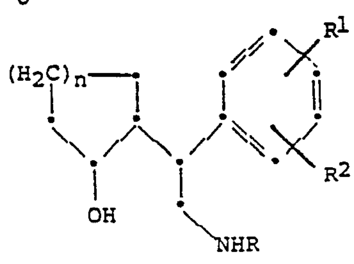

(II)

dans laquelle $R^1$, $R^2$ et n ont la signification donnée ci-dessus et R représente l'hydrogène ou un alkyle inférieur, ou di- ou mono-alkyle respectivement le groupe amino primaire ou secondaire, ou

b) dans un composé de formule générale

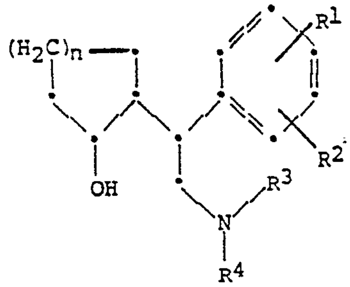

(Ic)

,

dans laquelle $R^1$, $R^2$, $R^3$, $R^4$ et n ont la signification donnée ci-dessus, on oxyde le groupe alcool secondaire, ou

c) dans un composé de formule générale

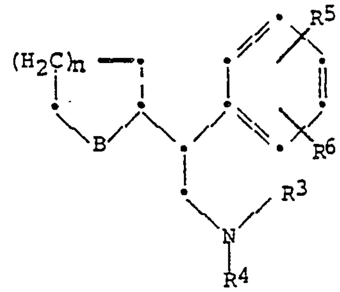

(III)

dans laquelle $R^3$, $R^4$, n et B ont la signification donnée ci-dessus, et l'un des restes $R^5$ et $R^6$ est un groupe hydroxyle protégé et l'autre est un hydrogène, halogène, trifluorométhyle, alcoxy inférieur, alkyle inférieur, nitro ou un groupe hydroxyle protégé, on élimine le(s) groupe(s) protecteur(s), ou

d) on nitre en position p du noyau phényle d'un composé de formule générale

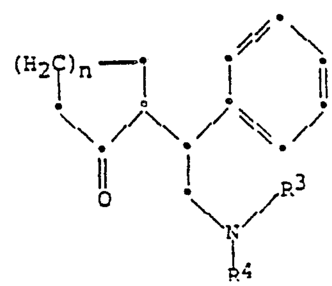

(IV)

dans laquelle $R^3$, $R^4$ et n ont la signification donnée ci-dessus et, si c'est souhaité,

e) on sépare un mélange obtenu de différents racémates en les racémates,

f) on fractionne un racémate obtenu en ses antipodes optiques et/ou

g) on transforme un composé de formule I obtenu en un sel d'addition d'acide pharmaceutiquement acceptable.

23. Procédé selon la revendication 22, dans lequel n représente le nombre 1, 2 ou 3.

24. Procédé selon la revendication 22 ou 23, caractérisé en ce qu'on prépare un composé avec la configuration relative représentée dans la formule Ia ou Ib ou les formes énantiomères unitaires optiques correspondantes :

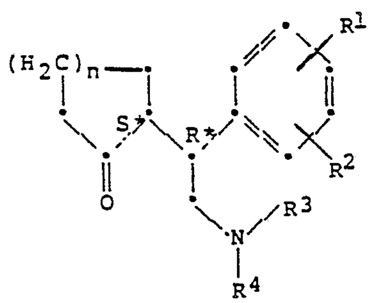

(Ia)

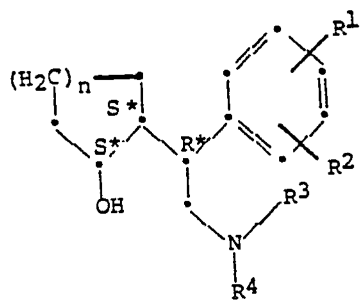

(Ib)

25. Procédé selon l'une des revendications 22-24, caractérisé en ce qu'on prépare un composé de formule I, dans laquelle soit $R^1$ est un halogène, alcoxy inférieur ou hydroxy et $R^2$ est l'hydrogène, soit $R^1$ et $R^2$ représentent tous les deux un halogène, alcoxy inférieur ou hydroxy.

26. Procédé selon l'une des revendications 22-25, caractérisé en ce qu'on prépare un composé de formule I, dans laquelle n est le nombre 2.

27. Procédé selon l'une des revendications 22-26, caractérisé en ce qu'on prépare un composé de formule I, dans laquelle B est le groupe —CHOH—.

28. Procédé selon l'une des revendications 22-27, caractérisé en ce qu'on prépare un composé de formule I, dans laquelle $R^3$ et $R^4$ représentent le méthyle.

29. Procédé selon la revendication 22 ou 23, caractérisé en ce qu'on prépare le rac-(1S*)-cis-2-/(R*)-4-chloro-α-[(diméthylamino) méthyl] benzyl/cyclohexanol.

30. Procédé selon la revendication 22 ou 23, caractérisé en ce qu'on prépare le rac-(1S*)-cis-2-/(R*)-α-[(diméthylamino) méthyl]-4-hydroxybenzyl/cyclohexanol.

31. Procédé selon la revendication 22 ou 23, caractérisé en ce qu'on prépare le (1S)-cis-2-/(R)-4-chloro-α-[(diméthylamino) méthyl] benzyl/cyclohexanol.

32. Procédé selon la revendication 22 ou 23, caractérisé en ce qu'on prépare le (1R)-cis-2-/(S)-4-chloro-α-[(diméthylamino) méthyl] benzyl/cyclohexanol.

33. Procédé selon la revendication 22 ou 23, caractérisé en ce qu'on prépare le rac-(1S*)-cis-2-/(R*)-3,4-dichloro-α-[(diméthylamino) méthyl] benzyl/cyclohexanol.

34. Procédé selon la revendication 22 ou 23, caractérisé en ce qu'on prépare le rac-(1S*)-cis-2-/(R*)-4-méthoxy-α-[(diméthylamino) méthyl] benzyl/cyclohexanol.

35. Procédé selon la revendication 22 ou 23, caractérisé en ce qu'on prépare le rac-(1S*)-cis-2-/(R*)-α-[(dimméthylamino) méthyl]-3,4-diméthoxybenzyl] cyclohexanol.

36. Procédé selon la revendication 22 ou 23, caractérisé en ce qu'on prépare le rac-(2S*)-2-/(R*)-4-chloro-α-[(diméthylamino) méthyl] benzyl/cyclohexanone.

37. Procédé selon la revendication 22 ou 23, caractérisé en ce qu'on prépare le (1S)-cis-2-/(R)-α-[(diméthylamino) méthyl]-4-hydroxybenzyl/cyclohexanol.

38. Médicament contenant un composé selon l'une des revendications 1-16 et un excipient thérapeutiquement inerte.

39. Utilisation d'un composé selon l'une des revendications 1-16, pour préparer un médicament efficace analgésique et antidépresseur.